# EUROPEAN PATENT SPECIFICATION

(11) **EP 3 439 689 B1**
(45) Date of publication and mention of the grant of the patent: **13.10.2021**
(21) Application number: 17719107.9
(22) Date of filing: 07.04.2017
(51) Int. Cl.: A61K 39/00, A61P 3/06, C07K 16/22

(54) **METHODS FOR TREATING HYPERLIPIDEMIA WITH AN ANGPTL8 INHIBITOR AND AN ANGPTL3 INHIBITOR**
VERFAHREN ZUR BEHANDLUNG VON HYPERLIPIDÄMIE MIT EINEM ANGPTL8-INHIBITOR UND EINEM ANGPTL3-INHIBITOR
MÉTHODES DE TRAITEMENT DE L'HYPERLIPIDÉMIE À L'AIDE D'UN INHIBITEUR D'ANGPTL8 ET D'UN INHIBITEUR D'ANGPTL3

(30) Priority: 08.04.2016 US 201662319980 P; 01.02.2017 US 201762453110 P
(43) Date of publication of application: 13.02.2019
(73) Proprietor: Regeneron Pharmaceuticals, Inc., Tarrytown, NY 10591 (US)
(72) Inventor: GROMADA, Jesper, Tarrytown, New York 10591 (US); GUSAROVA, Viktoria, Tarrytown, New York 10591 (US); MURPHY, Andrew J., Tarrytown, New York 10591 (US)
(74) Representative: J A Kemp LLP
(86) International application number: PCT/US2017/026679
(87) International publication number: WO 2017/177181

(56) References cited:
- WO-A1-2012/174178
- WO-A1-2017/027316
- REN ZHANG: "The ANGPTL3-4-8 model, a molecular mechanism for triglyceride trafficking", OPEN BIOLOGY, vol. 6, no. 4, 1 April 2016 (2016-04-01), page 150272, XP055387866, DOI: 10.1098/rsob.150272
- ZHIYAO FU ET AL: "A lipasin/Angptl8 monoclonal antibody lowers mouse serum triglycerides involving increased postprandial activity of the cardiac lipoprotein lipase", SCIENTIFIC REPORTS, vol. 5, no. 1, 21 December 2015 (2015-12-21), XP055360939, DOI: 10.1038/srep18502
- HANSON ROBERT L ET AL: "The Arg59Trp variant in ANGPTL8 (betatrophin) is associated with total and HDL-cholesterol in American Indians and Mexican Americans and differentially affects cleavage of ANGPTL3", MOLECULAR GENETICS AND METABOLISM, ACADEMIC PRESS, AMSTERDAM, NL, vol. 118, no. 2, 19 April 2016 (2016-04-19), pages 128-137, XP029548394, ISSN: 1096-7192, DOI: 10.1016/J.YMGME.2016.04.007
- JORGE F. HALLER ET AL: "ANGPTL8 requires ANGPTL3 to inhibit lipoprotein lipase and plasma triglyceride clearance", JOURNAL OF LIPID RESEARCH, vol. 58, no. 6, 1 June 2017 (2017-06-01), pages 1166-1173, XP055387875, US ISSN: 0022-2275, DOI: 10.1194/jlr.M075689

### SEQUENCE LISTING

<110> REGENERON PHARMACEUTICALS, INC.
<120> METHODS FOR TREATING HYPERLIPIDEMIA WITH AN ANGPTL8 INHIBITOR AND AN ANGPTL3 INHIBITOR
<130> 0431.25PCT
<140>
   <141>
<150> 62/453,110 <151> 2017-02-01
<150> 62/319,980 <151> 2016-04-08
<160> 19
<170> PatentIn version 3.5
<210> 1
   <211> 120
   <212> PRT
   <213> Artificial Sequence
<220>
   <221> source
   <223> /note="Description of Artificial Sequence: Synthetic polypeptide"
<220>
   <221> source
   <223> /note="HCVR"
<400> 1
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Asn His
               20                  25                  30
   Glu Met Asn Trp Val Arg Gln Ala Pro Gly Lys Gly Leu Glu Trp Val
           35                  40                  45
   Ser Tyr Ile Ser Ser Ser Gly Asn Thr Val Thr Tyr Ala Asp Phe Leu
       50                  55                  60
   Lys Gly Arg Phe Thr Ile Ser Arg Asp Asn Ala Lys Asn Ser Leu Phe
   65                  70                  75                  80
   Leu Gln Met Asn Ser Leu Arg Asp Glu Asp Thr Ala Val Tyr Tyr Cys
                   85                  90                  95
   Ala Arg Asp His Leu Ser Gly Thr Ser Pro Leu Ser Tyr Trp Gly Gln
               100                 105                 110
   Gly Thr Leu Val Thr Val Ser Ser
           115                 120
<210> 2
   <211> 8
   <212> PRT
   <213> Artificial Sequence
<220>
   <221> source
   <223> /note="Description of Artificial Sequence: Synthetic peptide"
<220>
   <221> source
   <223> /note="HCDR1"
<400> 2
   Gly Phe Thr Phe Asn Asn His Glu
   1               5
<210> 3
   <211> 8
   <212> PRT
   <213> Artificial Sequence
<220>
   <221> source
   <223> /note="Description of Artificial Sequence: Synthetic peptide"
<220>
   <221> source
   <223> /note="HCDR2"
<400> 3
   Ile Ser Ser Ser Gly Asn Thr Val
   1 5
<210> 4
   <211> 13
   <212> PRT
   <213> Artificial Sequence
<220>
   <221> source
   <223> /note="Description of Artificial Sequence: Synthetic peptide"
<220>
   <221> source
   <223> /note="HCDR3"
<400> 4
   Ala Arg Asp His Leu Ser Gly Thr Ser Pro Leu Ser Tyr
   1 5 10
<210> 5
   <211> 107
   <212> PRT
   <213> Artificial Sequence
<220>
   <221> source
   <223> /note="Description of Artificial Sequence: Synthetic polypeptide"
<220>
   <221> source
   <223> /note="LCVR"
<400> 5
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asp Ile Asn Asn Tyr
               20                  25                  30
   Leu Asn Trp Phe Gln Gln Lys Pro Gly Lys Ala Pro Lys Leu Leu Ile
           35                  40                  45
   Phe Asp Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Phe Cys Gln Gln Tyr Glu Asn Leu Pro Tyr
                   85                  90                  95
   Thr Phe Gly Gln Gly Thr Lys Leu Glu Ile Lys
               100                 105
<210> 6
   <211> 6
   <212> PRT
   <213> Artificial Sequence
<220>
   <221> source
   <223> /note="Description of Artificial Sequence: Synthetic peptide"
<220>
   <221> source
   <223> /note="LCDR1"
<400> 6
   Gln Asp Ile Asn Asn Tyr
   1               5
<210> 7
   <211> 3
   <212> PRT
   <213> Artificial Sequence
<220>
   <221> source
   <223> /note="Description of Artificial Sequence: Synthetic peptide"
<220>
   <221> source
   <223> /note="LCDR2"
<400> 7
   Asp Ala Ser
   1
<210> 8
   <211> 9
   <212> PRT
   <213> Artificial Sequence
<220>
   <221> source
   <223> /note="Description of Artificial Sequence: Synthetic peptide"
<220>
   <221> source
   <223> /note="LCDR3"
<400> 8
   Gln Gln Tyr Glu Asn Leu Pro Tyr Thr
   1 5
<210> 9
   <211> 413
   <212> PRT
   <213> Artificial Sequence
<220>
   <221> source
   <223> /note="Description of Artificial Sequence: Synthetic polypeptide"
<220>
   <221> source
   <223> /note="hANGPTL8-mFc aa 1-177: amino acids 22-198 of NP_061157.3 aa 178-413: GPG linker and mouse IgG2a Fc tag"
<400> 9
   Ala Pro Met Gly Gly Pro Glu Leu Ala Gln His Glu Glu Leu Thr Leu
   1 5 10 15
   Leu Phe His Gly Thr Leu Gln Leu Gly Gln Ala Leu Asn Gly Val Tyr
   20 25 30
   Arg Thr Thr Glu Gly Arg Leu Thr Lys Ala Arg Asn Ser Leu Gly Leu
   35 40 45
   Tyr Gly Arg Thr Ile Glu Leu Leu Gly Gln Glu Val Ser Arg Gly Arg
   50 55 60
   Asp Ala Ala Gln Glu Leu Arg Ala Ser Leu Leu Glu Thr Gln Met Glu
   65 70 75 80
   Glu Asp Ile Leu Gln Leu Gln Ala Glu Ala Thr Ala Glu Val Leu Gly
   85 90 95
   Glu Val Ala Gln Ala Gln Lys Val Leu Arg Asp Ser Val Gln Arg Leu
   100 105 110
   Glu Val Gln Leu Arg Ser Ala Trp Leu Gly Pro Ala Tyr Arg Glu Phe
   115 120 125
   Glu Val Leu Lys Ala His Ala Asp Lys Gln Ser His Ile Leu Trp Ala
   130 135 140
   Leu Thr Gly His Val Gln Arg Gln Arg Arg Glu Met Val Ala Gln Gln
   145 150 155 160
   His Arg Leu Arg Gln Ile Gln Glu Arg Leu His Thr Ala Ala Leu Pro
   165 170 175
   Ala Gly Pro Gly Glu Pro Arg Gly Pro Thr Ile Lys Pro Cys Pro Pro
   180 185 190
   Cys Lys Cys Pro Ala Pro Asn Leu Leu Gly Gly Pro Ser Val Phe Ile
   195 200 205
   Phe Pro Pro Lys Ile Lys Asp Val Leu Met Ile Ser Leu Ser Pro Ile
   210 215 220
   Val Thr Cys Val Val Val Asp Val Ser Glu Asp Asp Pro Asp Val Gln
   225 230 235 240
   Ile Ser Trp Phe Val Asn Asn Val Glu Val His Thr Ala Gln Thr Gln
   245 250 255
   Thr His Arg Glu Asp Tyr Asn Ser Thr Leu Arg Val Val Ser Ala Leu
   260 265 270
   Pro Ile Gln His Gln Asp Trp Met Ser Gly Lys Glu Phe Lys Cys Lys
   275 280 285
   Val Asn Asn Lys Asp Leu Pro Ala Pro Ile Glu Arg Thr Ile Ser Lys
       290                 295                 300
   Pro Lys Gly Ser Val Arg Ala Pro Gln Val Tyr Val Leu Pro Pro Pro
   305                 310                 315                 320
   Glu Glu Glu Met Thr Lys Lys Gln Val Thr Leu Thr Cys Met Val Thr
                   325                 330                 335
   Asp Phe Met Pro Glu Asp Ile Tyr Val Glu Trp Thr Asn Asn Gly Lys
               340                 345                 350
   Thr Glu Leu Asn Tyr Lys Asn Thr Glu Pro Val Leu Asp Ser Asp Gly
           355                 360                 365
   Ser Tyr Phe Met Tyr Ser Lys Leu Arg Val Glu Lys Lys Asn Trp Val
       370                 375                 380
   Glu Arg Asn Ser Tyr Ser Cys Ser Val Val His Glu Gly Leu His Asn
   385                 390                 395                 400
   His His Thr Thr Lys Ser Phe Ser Arg Thr Pro Gly Lys
                   405                 410
<210> 10
   <211> 126
   <212> PRT
   <213> Artificial Sequence
<220>
   <221> source
   <223> /note="Description of Artificial Sequence: Synthetic polypeptide"
<220>
   <221> source
   <223> /note="Evinacumab HCVR"
<400> 10
   Glu Val Gln Leu Val Glu Ser Gly Gly Gly Val Ile Gln Pro Gly Gly
   1               5                   10                  15
   Ser Leu Arg Leu Ser Cys Ala Ala Ser Gly Phe Thr Phe Asp Asp Tyr
               20                  25                  30
   Ala Met Asn Trp Val Arg Gln Gly Pro Gly Lys Gly Leu Glu Trp Val
           35                  40                  45
   Ser Ala Ile Ser Gly Asp Gly Gly Ser Thr Tyr Tyr Ala Asp Ser Val
       50                  55                  60
   Lys Gly Arg Phe Thr Ile Ser Arg Asp Asn Ser Lys Asn Ser Leu Tyr
   65                  70                  75                  80
   Leu Gln Met Asn Ser Leu Arg Ala Glu Asp Thr Ala Phe Phe Tyr Cys
                   85                  90                  95
   Ala Lys Asp Leu Arg Asn Thr Ile Phe Gly Val Val Ile Pro Asp Ala
               100                 105                 110
   Phe Asp Ile Trp Gly Gln Gly Thr Met Val Thr Val Ser Ser
           115                 120                 125
<210> 11
   <211> 8
   <212> PRT
   <213> Artificial Sequence
<220>
   <221> source
   <223> /note="Description of Artificial Sequence: Synthetic peptide"
<220>
   <221> source
   <223> /note="Evinacumab HCDR1"
<400> 11
   Gly Phe Thr Phe Asp Asp Tyr Ala
   1               5
<210> 12
   <211> 8
   <212> PRT
   <213> Artificial Sequence
<220>
   <221> source
   <223> /note="Description of Artificial Sequence: Synthetic peptide"
<220>
   <221> source
   <223> /note="Evinacumab HCDR2"
<400> 12
   Ile Ser Gly Asp Gly Gly Ser Thr
   1 5
<210> 13
   <211> 19
   <212> PRT
   <213> Artificial Sequence
<220>
   <221> source
   <223> /note="Description of Artificial Sequence: Synthetic peptide"
<220>
   <221> source
   <223> /note="Evinacumab HCDR3"
<400> 13
   Ala Lys Asp Leu Arg Asn Thr Ile Phe Gly Val Val Ile Pro Asp Ala
   1 5 10 15
   Phe Asp Ile
<210> 14
   <211> 108
   <212> PRT
   <213> Artificial Sequence
<220>
   <221> source
   <223> /note="Description of Artificial Sequence: Synthetic polypeptide"
<220>
   <221> source
   <223> /note="Evinacumab LCVR"
<400> 14
   Asp Ile Gln Met Thr Gln Ser Pro Ser Thr Leu Ser Ala Ser Val Gly
   1 5 10 15
   Asp Arg Val Thr Ile Thr Cys Arg Ala Ser Gln Ser Ile Arg Ser Trp
   20 25 30
   Leu Ala Trp Tyr Gln Gln Lys Pro Gly Lys Ala Pro Lys Leu Leu Ile
   35 40 45
   Tyr Lys Ala Ser Ser Leu Glu Ser Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Asp Asp Phe Ala Thr Tyr Tyr Cys Gln Gln Tyr Asn Ser Tyr Ser Tyr
   85 90 95
   Thr Phe Gly Gln Gly Thr Lys Leu Glu Ile Lys Arg
   100 105
<210> 15
   <211> 6
   <212> PRT
   <213> Artificial Sequence
<220>
   <221> source
   <223> /note="Description of Artificial Sequence: Synthetic peptide"
<220>
   <221> source
   <223> /note="Evinacumab LCDR1"
<400> 15
   Gln Ser Ile Arg Ser Trp
   1 5
<210> 16
   <211> 3
   <212> PRT
   <213> Artificial Sequence
<220>
   <221> source
   <223> /note="Description of Artificial Sequence: Synthetic peptide"
<220>
   <221> source
   <223> /note="Evinacumab LCDR2"
<400> 16
   Lys Ala Ser
   1
<210> 17
   <211> 9
   <212> PRT
   <213> Artificial Sequence
<220>
   <221> source
   <223> /note="Description of Artificial Sequence: Synthetic peptide"
<220>
   <221> source
   <223> /note="Evinacumab LCDR3"
<400> 17
   Gln Gln Tyr Asn Ser Tyr Ser Tyr Thr
   1 5
<210> 18
   <211> 460
   <212> PRT
   <213> Artificial Sequence
<220>
   <221> source
   <223> /note="Description of Artificial Sequence: Synthetic polypeptide"
<220>
   <221> source
   <223> /note="hANGPTL3"
<400> 18
   Met Phe Thr Ile Lys Leu Leu Leu Phe Ile Val Pro Leu Val Ile Ser
   1               5                   10                  15
   Ser Arg Ile Asp Gln Asp Asn Ser Ser Phe Asp Ser Leu Ser Pro Glu
               20                  25                  30
   Pro Lys Ser Arg Phe Ala Met Leu Asp Asp Val Lys Ile Leu Ala Asn
           35                  40                  45
   Gly Leu Leu Gln Leu Gly His Gly Leu Lys Asp Phe Val His Lys Thr
       50                  55                  60
   Lys Gly Gln Ile Asn Asp Ile Phe Gln Lys Leu Asn Ile Phe Asp Gln
   65                  70                  75                  80
   Ser Phe Tyr Asp Leu Ser Leu Gln Thr Ser Glu Ile Lys Glu Glu Glu
                   85                  90                  95
   Lys Glu Leu Arg Arg Thr Thr Tyr Lys Leu Gln Val Lys Asn Glu Glu
               100                 105                 110
   Val Lys Asn Met Ser Leu Glu Leu Asn Ser Lys Leu Glu Ser Leu Leu
           115                 120                 125
   Glu Glu Lys Ile Leu Leu Gln Gln Lys Val Lys Tyr Leu Glu Glu Gln
       130                 135                 140
   Leu Thr Asn Leu Ile Gln Asn Gln Pro Glu Thr Pro Glu His Pro Glu
   145                 150                 155                 160
   Val Thr Ser Leu Lys Thr Phe Val Glu Lys Gln Asp Asn Ser Ile Lys
                   165                 170                 175
   Asp Leu Leu Gln Thr Val Glu Asp Gln Tyr Lys Gln Leu Asn Gln Gln
               180                 185                 190
   His Ser Gln Ile Lys Glu Ile Glu Asn Gln Leu Arg Arg Thr Ser Ile
           195                 200                 205
   Gln Glu Pro Thr Glu Ile Ser Leu Ser Ser Lys Pro Arg Ala Pro Arg
       210                 215                 220
   Thr Thr Pro Phe Leu Gln Leu Asn Glu Ile Arg Asn Val Lys His Asp
   225                 230                 235                 240
   Gly Ile Pro Ala Glu Cys Thr Thr Ile Tyr Asn Arg Gly Glu His Thr
                   245                 250                 255
   Ser Gly Met Tyr Ala Ile Arg Pro Ser Asn Ser Gln Val Phe His Val
               260                 265                 270
   Tyr Cys Asp Val Ile Ser Gly Ser Pro Trp Thr Leu Ile Gln His Arg
           275                 280                 285
   Ile Asp Gly Ser Gln Asn Phe Asn Glu Thr Trp Glu Asn Tyr Lys Tyr
       290                 295                 300
   Gly Phe Gly Arg Leu Asp Gly Glu Phe Trp Leu Gly Leu Glu Lys Ile
   305                 310                 315                 320
   Tyr Ser Ile Val Lys Gln Ser Asn Tyr Val Leu Arg Ile Glu Leu Glu
                   325                 330                 335
   Asp Trp Lys Asp Asn Lys His Tyr Ile Glu Tyr Ser Phe Tyr Leu Gly
               340                 345                 350
   Asn His Glu Thr Asn Tyr Thr Leu His Leu Val Ala Ile Thr Gly Asn
           355                 360                 365
   Val Pro Asn Ala Ile Pro Glu Asn Lys Asp Leu Val Phe Ser Thr Trp
       370                 375                 380
   Asp His Lys Ala Lys Gly His Phe Asn Cys Pro Glu Gly Tyr Ser Gly
   385                 390                 395                 400
   Gly Trp Trp Trp His Asp Glu Cys Gly Glu Asn Asn Leu Asn Gly Lys
                   405                 410                 415
   Tyr Asn Lys Pro Arg Ala Lys Ser Lys Pro Glu Arg Arg Arg Gly Leu
               420                 425                 430
   Ser Trp Lys Ser Gln Asn Gly Arg Leu Tyr Ser Ile Lys Ser Thr Lys
           435                 440                 445
   Met Leu Ile His Pro Thr Asp Ser Glu Ser Phe Glu
       450                 455                 460
<210> 19
   <211> 198
   <212> PRT
   <213> Homo sapiens
<400> 19
   Met Pro Val Pro Ala Leu Cys Leu Leu Trp Ala Leu Ala Met Val Thr
   1               5                   10                  15
   Arg Pro Ala Ser Ala Ala Pro Met Gly Gly Pro Glu Leu Ala Gln His
               20                  25                  30
   Glu Glu Leu Thr Leu Leu Phe His Gly Thr Leu Gln Leu Gly Gln Ala
           35                  40                  45
   Leu Asn Gly Val Tyr Arg Thr Thr Glu Gly Arg Leu Thr Lys Ala Arg
       50                  55                  60
   Asn Ser Leu Gly Leu Tyr Gly Arg Thr Ile Glu Leu Leu Gly Gln Glu
   65                  70                  75                  80
   Val Ser Arg Gly Arg Asp Ala Ala Gln Glu Leu Arg Ala Ser Leu Leu
                   85                  90                  95
   Glu Thr Gln Met Glu Glu Asp Ile Leu Gln Leu Gln Ala Glu Ala Thr
               100                 105                 110
   Ala Glu Val Leu Gly Glu Val Ala Gln Ala Gln Lys Val Leu Arg Asp
           115                 120                 125
   Ser Val Gln Arg Leu Glu Val Gln Leu Arg Ser Ala Trp Leu Gly Pro
       130                 135                 140
   Ala Tyr Arg Glu Phe Glu Val Leu Lys Ala His Ala Asp Lys Gln Ser
   145                 150                 155                 160
   His Ile Leu Trp Ala Leu Thr Gly His Val Gln Arg Gln Arg Arg Glu
                   165                 170                 175
   Met Val Ala Gln Gln His Arg Leu Arg Gln Ile Gln Glu Arg Leu His
               180                 185                 190
   Thr Ala Ala Leu Pro Ala
           195

## Description

### FIELD OF THE INVENTION

The present invention relates to the field of therapeutic treatments of diseases and disorders, which are associated with elevated levels of lipids and lipoproteins. More specifically, the invention relates to the use of an angiopoietin-like protein 8 (ANGPTL8) inhibitor in combination with an inhibitor of angiopoietin-like protein 3 (ANGPTL3) to treat patients with hypercholesterolemia and related conditions.

### BACKGROUND

Hyperlipidemia is a general term that encompasses diseases and disorders characterized by or associated with elevated levels of lipids and/or lipoproteins in the blood. Hyperlipidemias include hypercholesterolemia, hypertriglyceridemia, combined hyperlipidemia, and elevated lipoprotein a (Lp(a)). A particular prevalent form of hyperlipidemia in many populations is hypercholesterolemia.

Hypercholesterolemia, particularly an increase in low-density lipoprotein (LDL) cholesterol (LDL-C) levels, constitutes a major risk for the development of atherosclerosis and coronary heart disease (CHD) (Sharrett et al., 2001, Circulation 104:1108-1113). Low-density lipoprotein cholesterol is identified as the primary target of cholesterol lowering therapy and is accepted as a valid surrogate therapeutic endpoint. Numerous studies have demonstrated that reducing LDL-C levels reduces the risk of CHD with a strong direct relationship between LDL-C levels and CHD events; for each 1 mmol/L (-40 mg/dL) reduction in LDL-C, cardiovascular disease (CVD) mortality and morbidity is lowered by 22%. Greater reductions in LDL-C produce greater reduction in events, and comparative data of intensive versus standard statin treatment suggest that the lower the LDL-C level, the greater the benefit in patients at very high cardiovascular (CV) risk.

Familial hypercholesterolemia (FH) is an inherited disorder of lipid metabolism that predisposes a person to premature severe cardiovascular disease (CVD) (Kolansky et al., (2008), Am J Cardiology,102(11):1438-1443). FH can be either an autosomal dominant or an autosomal recessive disease that results from mutations in the low density lipoprotein receptor (LDLR), or in at least 3 different genes that code for proteins involved in hepatic clearance of LDL-C can cause FH. Examples of such defects include mutations in the gene coding for the LDL receptor (LDLR) that removes LDL-C from the circulation, and in the gene for apolipoprotein (Apo) B, which is the major protein of the LDL particle. In all cases, FH is characterized by an accumulation of LDL-C in the plasma from birth and subsequent development of tendon xanthomas, xanthelasmas, atheromata, and CVD. FH can be classified as either heterozygous FH (heFH) or homozygous FH (hoFH) depending on whether the individual has a genetic defect in one (heterozygous) or both (homozygous) copies of the implicated gene.

Current LDL-C-lowering medications include statins, cholesterol absorption inhibitors, fibrates, niacin, and bile acid sequestrants. Statins are a commonly prescribed treatment for LDL-C lowering. However, despite the availability of such lipid-lowering therapies, many high-risk patients fail to reach their guideline target LDL-C level (Gitt et al., 2010, Clin Res Cardiol 99(11):723-733). For patients who are still unable to achieve guideline target level for LDL-C, despite available lipid-modifying therapy (LMT), mechanical removal of LDL-C by lipoprotein apheresis (e.g., LDL apheresis) is sometimes prescribed.

However, patients who are not at LDL-C goal despite receiving an optimized LMT regimen, would greatly benefit from alternative LDL-C lowering therapies, or through use of a combination of therapeutic agents, such as the agents and regimens described herein.

Fu et al (2015) Scientific Reports, 5, 1-9 discloses generation of five monoclonal lipasin antibodies, among which one lowered the serum triglyceride level when injected into mice. Zhang (2016) Open Biology, 6, 150272 reviews the ANGPTL3-4-8 model as a molecular mechanism for triglyceride trafficking.

### BRIEF SUMMARY OF THE INVENTION

The present invention provides a therapeutically effective amount of an inhibitor of angiopoietin-like protein 8 (ANGPTL8) for use in a method of treating a patient with hyperlipidemia in combination with a therapeutically effective amount of an inhibitor of angiopoietin-like protein 3 (ANGPTL3). The present invention also provides a therapeutically effective amount of an inhibitor of ANGPTL3 for use in a method of treating a patient with hyperlipidemia in combination with a therapeutically effective amount of an inhibitor of ANGPTL8.

In one embodiment, the hyperlipidemia is familial hyperlipidemia or acquired hyperlipidemia.

In one embodiment, the hyperlipidemia is selected from the group consisting of hyperlipoproteinemia, hypercholesterolemia, hypertriglyceridemia, or hyperchylomicronemia.

In one embodiment, the familial hyperlipidemia is hypercholesterolemia selected from the group consisting of heterozygous familial hypercholesterolemia (HeFH) and homozygous familial hypercholesterolemia (HoFH).

In one embodiment, the acquired hyperlipidemia is the result of excessive drinking of alcohol, obesity, side effects of medications (e.g. hormones or steroids), diabetes, kidney disease, underactive thyroid gland, or pregnancy.

In the invention the ANGPTL8 inhibitor is an antibody, or an antigen-binding fragment thereof, that binds specifically to ANGPTL8.

In one aspect of the disclosure, the antibody, or antigen-binding fragment thereof that binds specifically to ANGPTL8 comprises the complementarity determining regions (CDRs) of a heavy chain variable (HCVR) having the amino acid sequence of SEQ ID NO: 1 and the CDRs of a light chain variable region (LCVR) of SEQ ID NO: 5.

In the invention, the antibody, or antigen-binding fragment thereof that binds specifically to ANGPTL8 comprises a heavy chain CDR1 (HCDR1) having the amino acid sequence of SEQ ID NO: 2, a HCDR2 having the amino acid sequence of SEQ ID NO: 3, a HCDR3 having the amino acid sequence of SEQ ID NO: 4, a light chain CDR1 (LCDR1) having the amino acid sequence of SEQ ID NO: 6, a LCDR2 having the amino acid sequence of SEQ ID NO: 7, and a LCDR3 having the amino acid sequence of SEQ ID NO: 8.

In one embodiment, the antibody, or antigen-binding fragment thereof that binds specifically to ANGPTL8 comprises a HCVR having the amino acid sequence of SEQ ID NO: 1 and a LCVR having the amino acid sequence of SEQ ID NO: 5.

In one embodiment, the ANGPTL8 antibody is administered to the patient subcutaneously or intravenously.

In the invention, the ANGPTL3 inhibitor is an antibody, or an antigen-binding fragment thereof, that binds specifically to ANGPTL3.

In one embodiment, the ANGPTL3 antibody is evinacumab.

In one aspect of the disclosure, the antibody, or antigen-binding fragment thereof that binds specifically to ANGPTL3 comprises the complementarity determining regions (CDRs) of a heavy chain variable (HCVR) having the amino acid sequence of SEQ ID NO: 10 and the CDRs of a light chain variable region (LCVR) of SEQ ID NO: 14.

In the invention, the antibody, or antigen-binding fragment thereof that binds specifically to ANGTL3 comprises a heavy chain CDR1 (HCDR1) having the amino acid sequence of SEQ ID NO: 11, a HCDR2 having the amino acid sequence of SEQ ID NO: 12, a HCDR3 having the amino acid sequence of SEQ ID NO: 13, a light chain CDR1 (LCDR1) having the amino acid sequence of SEQ ID NO: 15, a LCDR2 having the amino acid sequence of SEQ ID NO: 16, and a LCDR3 having the amino acid sequence of SEQ ID NO: 17.

In one embodiment, the antibody, or antigen-binding fragment thereof that binds specifically to ANGPTL3 comprises a HCVR having the amino acid sequence of SEQ ID NO: 10 and a LCVR having the amino acid sequence of SEQ ID NO: 14.

In one embodiment, the ANGPTL3 antibody is administered to the patient subcutaneously or intravenously.

The disclosure also provides for methods of reducing the level of at least one lipid parameter in a patient suffering from a disorder or condition characterized in part by elevated levels of lipids or lipoproteins, the method comprising administering to the patient a therapeutically effective amount of a combination of an angiopoietin-like protein 8 (ANGPTL8) inhibitor and an inhibitor of angiopoietin-like protein 3 (ANGPTL3).

In one aspect, the disorder or condition is hyperlipidemia selected from the group consisting of familial hyperlipidemia and acquired hyperlipidemia.

In one aspect, the disorder or condition is hyperlipidemia selected from the group consisting of hyperlipoproteinemia, hypercholesterolemia, hypertriglyceridemia, or hyperchylomicronemia.

In one aspect, the familial hyperlipidemia is hypercholesterolemia selected from the group consisting of heterozygous familial hypercholesterolemia (HeFH) and homozygous familial hypercholesterolemia (HoFH).

In one aspect, the acquired hyperlipidemia is the result of excessive drinking of alcohol, obesity, side effects of medications (e.g. hormones or steroids), diabetes, kidney disease, underactive thyroid gland, or pregnancy.

In one aspect, the hypertriglyceridemia treatable includes familial combined hyperlipidemia, familial dysbetalipoproteinemia, familial hypertriglyceridemia, severe chylomicronemia and familial lipoprotein lipase deficiency.

In one aspect, the ANGPTL8 inhibitor is an antibody, or an antigen-binding fragment thereof, that binds specifically to ANGPTL8.

In one aspect, the antibody, or antigen-binding fragment thereof that binds specifically to ANGPTL8 comprises the complementarity determining regions (CDRs) of a heavy chain variable (HCVR) having the amino acid sequence of SEQ ID NO: 1 and the CDRs of a light chain variable region (LCVR) of SEQ ID NO: 5.

In one aspect, the antibody, or antigen-binding fragment thereof that binds specifically to ANGPTL8 comprises a heavy chain CDR1 (HCDR1) having the amino acid sequence of SEQ ID NO: 2, a HCDR2 having the amino acid sequence of SEQ ID NO: 3, a HCDR3 having the amino acid sequence of SEQ ID NO: 4, a light chain CDR1 (LCDR1) having the amino acid sequence of SEQ ID NO: 6, a LCDR2 having the amino acid sequence of SEQ ID NO: 7, and a LCDR3 having the amino acid sequence of SEQ ID NO: 8.

In one aspect, the antibody, or antigen-binding fragment thereof that binds specifically to ANGPTL8 comprises a HCVR having the amino acid sequence of SEQ ID NO: 1 and a LCVR having the amino acid sequence of SEQ ID NO: 5.

In one aspect, the ANGPTL8 antibody is administered to the patient subcutaneously or intravenously.

In one aspect, the ANGPTL3 inhibitor is an antibody, or an antigen-binding fragment thereof, that binds specifically to ANGPTL3.

In one aspect, the ANGPTL3 antibody is evinacumab.

In one aspect, the antibody, or antigen-binding fragment thereof that binds specifically to ANGPTL3 comprises the complementarity determining regions (CDRs) of a heavy chain variable (HCVR) having the amino acid sequence of SEQ ID NO: 10 and the CDRs of a light chain variable region (LCVR) of SEQ ID NO: 14.

In one aspect, the antibody, or antigen-binding fragment thereof that binds specifically to ANGTL3 comprises a heavy chain CDR1 (HCDR1) having the amino acid sequence of SEQ ID NO: 11, a HCDR2 having the amino acid sequence of SEQ ID NO: 12, a HCDR3 having the amino acid sequence of SEQ ID NO: 13, a light chain CDR1 (LCDR1) having the amino acid sequence of SEQ ID NO: 15, a LCDR2 having the amino acid sequence of SEQ ID NO: 16, and a LCDR3 having the amino acid sequence of SEQ ID NO: 17.

In one aspect, the antibody, or antigen-binding fragment thereof that binds specifically to ANGPTL3 comprises a HCVR having the amino acid sequence of SEQ ID NO: 10 and a LCVR having the amino acid sequence of SEQ ID NO: 14.

In one aspect, the ANGPTL3 antibody is administered to the patient subcutaneously or intravenously.

In one embodiment of the invention, the ANGPTL8 inhibitor and the ANGPTL3 inhibitor are administered concurrently or sequentially.

In one embodiment of the invention, the ANGPTL8 inhibitor and the ANGPTL3 inhibitor are administered at therapeutically effective concentrations in separate pharmaceutical compositions or are co-formulated in one pharmaceutical composition.

In some embodiments the invention relates to treating hyperlipidemia in patients who are non-responsive to, inadequately controlled by, or intolerant to treatment with a standard lipid modifying therapy. The therapeutic methods result in a lowering of serum lipoprotein levels to a normal and acceptable range and as such, may act to reduce the risk of development of atherosclerosis, or coronary heart disease.

In one embodiment, the invention relates to a method of treating a patient suffering from hypercholesterolemia, wherein the patient is non-responsive to, inadequately controlled by, or intolerant to treatment with a standard lipid modifying therapy, the method comprising treating the patient with a combination of an angiopoietin-like protein 8 (ANGPTL8) inhibitor and an inhibitor of angiopoietin-like protein 3 (ANGPTL3).

In one embodiment, the invention provides administering one or more doses of an ANGPTL8 inhibitor in combination with one or more doses of an ANGPTL3 inhibitor to a patient who is being treated, or has been treated with a standard lipid modifying therapy, but has not responded to such therapy. Administration of a combination of an ANGPTL8 inhibitor with an ANGPTL3 inhibitor to the patient results in lowering the level of at least one lipoprotein in the serum of the patient and consequently reduces or eliminates the need for treatment with the standard lipid lowering therapy by the patient.

The invention may comprise selecting a patient with hypercholesterolemia who is being treated, or has been treated with a standard lipid lowering therapy and who is non-responsive to, inadequately controlled by, or intolerant to, such therapy and administering one or more doses of a ANGPTL8 antibody in combination with one or more doses of an ANGPTL3 antibody to the patient, thereby lowering the level of at least one lipoprotein in the serum of the patient and consequently replacing the use of the standard lipid modifying therapy with the combination therapy of a ANGPTL8 antibody plus an ANGPTL3 antibody to achieve a target lipoprotein level.

Patients who are treated or treatable in the invention include, e.g., patients with hypercholesterolemia, including patients with familial hypercholesterolemia (FH). In certain embodiments, the patients who are treated or treatable by the methods are patients who are diagnosed with (or otherwise known to have), homozygous FH (hoFH) or heterozygous FH (heFH), or at risk for developing abnormally high lipid and/or lipoprotein levels associated with homozygous FH (hoFH) or heterozygous FH (heFH).

The present invention also provides pharmaceutical compositions comprising an ANGPTL8 inhibitor and an ANGPTL3 inhibitor for use in treating a patient who is non-responsive to, inadequately controlled by, or intolerant to treatment with a standard lipid modifying therapy, such as a statin. The statin may be selected from the group consisting of atorvastatin (LIPITOR®), pitavastatin (LIVALO®), lovastatin (MEVACOR®), simvastatin (ZOCOR®), pravastatin (PRAVACHOL®) fluvastatin (LESCOL®) and rosuvastatin (CRESTOR®). Other standard lipid lowering agents that may be used in patients suffering from hypercholesterolemia, include, but are not limited to, fibrates, niacin, bile acid sequestrants, ezetimibe (ZETIA®), lomitapide (JUXTAPID®), phytosterols, orlistat (XENICAL®).

Exemplary ANGPTL8 inhibitors, or ANGPTL3 inhibitors that may be used in the context of the methods of the present disclosure include, e.g., anti-ANGPTL8 or anti-ANGPTL3 antibodies, small molecule inhibitors, and scaffold-based, i.e. ANGPTL8-binding molecules, or ANGPTL3-binding molecules.

In certain embodiments, it is envisioned that the use of the combination of the ANGPTL8 inhibitor with the ANGPTL3 inhibitor may be sufficiently effective at lowering serum lipid and/or lipoprotein levels, such that the dose of the standard lipid modifying therapy may be reduced to eliminate any untoward effects, or it may be eliminated altogether.

In the invention, the administration of the ANGPTL8 antibody in combination with the ANGPTL3 antibody results in an additive effect on lowering the blood level of triglycerides and total cholesterol.

In one aspect of the disclosure, the administration of the ANGPTL8 antibody in combination with the ANGPTL3 antibody results in a synergistic effect on lowering the blood level of triglycerides and total cholesterol.

The administration of the ANGPTL8 antibody in combination with the ANGPTL3 antibody may result in lowering one or more of the following parameters:
(a) a reduction in serum total cholesterol (TC) level; or
(b) a reduction in serum triglycerides (TG);
wherein the reduction of (a), and/or (b) are determined relative to the patient's serum TC level, or serum triglyceride levels prior to, or at the time of initiation of treatment with the combination of the ANGPTL8 inhibitor and the ANGPTL3 inhibitor.

In one embodiment, the invention is directed to a pharmaceutical composition for treating a patient suffering from hyperlipidemia, wherein the composition comprises a therapeutically effective amount of a combination of an angiopoietin-like protein 8 (ANGPTL8) inhibitor as defined in the claims and an inhibitor of angiopoietin-like protein 3 (ANGPTL3) as defined in the claims. Other embodiments of the present invention will become apparent from a review of the ensuing detailed description.

### BRIEF DESCRIPTION OF THE FIGURES

**Figure 1** shows the effect of H4H1276S (also referred to as evinacumab), an anti-ANGPTL3 antibody, on triglyceride levels in ANGPTL8 knockout (KO) mice or in wild type (WT) mice at day 2 or 8 after administration.
**Figure 2** shows the effect of H4H1276S on total cholesterol levels in ANGPTL8 knockout mice or in wild type mice at day 2 or 8 after administration.
**Figure 3** shows the effect of H4H1276S and H4H15341P (also referred to as "H4H15341", an anti-ANGPTL8 antibody) on triglyceride levels in humanized ANGPTL8 mice on days 1, 4, 7 and 14 after administration.
**Figure 4** shows the effect of H4H1276S and H4H15341P (an anti-ANGPTL8 antibody) on total cholesterol levels in humanized ANGPTL8 mice on days 1, 4, 7 and 14 after administration.
**Figure 5** shows the results of anti-ANGPTL8 monoclonal antibodies epitope mapping: PEPSCAN ELISA was performed to examine the binding of anti-ANGPTL8 antibodies to 15-amino acid long peptides with 14 amino acid overlap, spanning mature ANGPTL8.

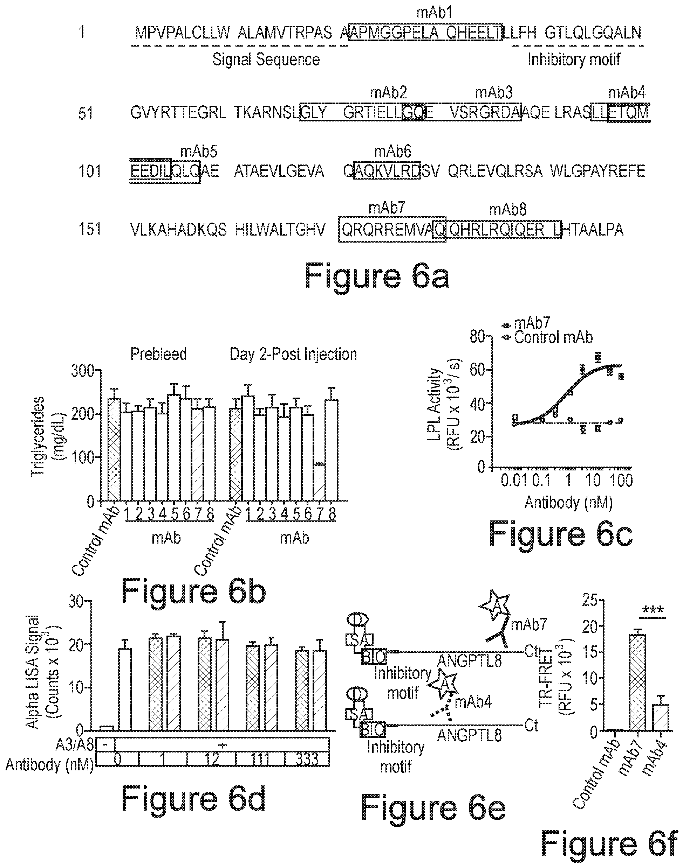

**Figures 6a, 6b, 6c, 6d, 6e, and 6f** show ANGPTL8 antibody reverses ANGPTL3:ANGPTL8-mediated LPL inhibition by steric interference of LPL with ANGPTL8 inhibitory motif. (Figure 6a) ANGPTL8 amino acid sequence (SEQ ID NO:19) depicting epitopes of eight monoclonal antibodies raised against human ANGPTL8. (Figure 6b) Serum triglycerides of humanized ANGPTL8 mice, 7 days before (pre-bleed) and 2 days after a single injection of 10 mg/ml of the eight monoclonal antibodies with epitopes shown in (Figure 6a). (Figure 6c) LPL activity measured in cell media from HEK283T cells co-transfected with ANGPTL3 and ANGPTL8 were treated with increased concentration of anti-ANGPTL8 blocking antibody (mAb7) or control antibody. (Figure 6d) AlphaLISA assay to determine interaction between ANGPTL3 and ANGPTL8 co-expressed in CHO-K1 cells with no or increasing concentrations of either anti-ANGPTL8 blocking antibody (mAb7) or control antibody. (Figure 6e) Scheme of the TR-FRET assay with donor dye at the N-terminus of ANGPTL8, near its inhibitory motif, and blocking and non-blocking antibodies labeled with acceptor dye. (Figure 6f) Energy transfer measured by TR-FRET assay between the N-terminus labeled ANGPTL8 and control antibody, anti-ANGPTL8 blocking antibody (mAb7), or non-blocking anti-ANGPTL8 (mAb4) (n=3; ****p*<0.0001). The experiment was repeated 3 times with the similar result. Abbreviations: Bio, biotin; SA, streptavidin, mAb, monoclonal antibody; D, donor dye; A, acceptor dye.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (e.g., 99.1, 99.2, 99.3, 99.4, etc.).

Although any methods and materials similar or equivalent to those described herein can be used in the practice of the present invention, the preferred methods and materials are now described.

### Methods for Treating Hyperlipidemias

The present invention relates generally to methods and compositions for reducing lipoprotein levels, particularly triglycerides and total cholesterol, in patients suffering from hyperlipidemia, by administering a combination of an inhibitor of ANGPTL8 with an inhibitor of ANGPTL3 to a patient in need of such therapy. In certain aspects, the combination of inhibitors is used to treat hypercholesterolemia, or hypertriglyceridemia, in patients who are non-responsive to, inadequately controlled by, or intolerant to standard lipid modifying therapies (e.g. a statin). In certain aspects of the disclosure, treatment with an ANGPTL8 inhibitor in combination with an ANGPTL3 inhibitor may serve to lower the levels of lipoproteins in these patients to an acceptable range, thereby lowering their risk for development of atherosclerosis, stroke and other cardiovascular diseases. In certain embodiments, the methods described may be used to treat patients suffering from hypercholesterolemia, including heterozygous familial hypercholesterolemia (heFH) and/or homozygous familial hypercholesterolemia (hoFH), in the event that these patients are non-responsive to, inadequately controlled by, or intolerant to standard lipid modifying therapies. In certain embodiment, the methods described may be used to treat patients suffering from, or at risk for developing atherosclerosis, coronary artery disease, diabetes, obesity, pancreatitis, or metabolic syndrome.

As used herein, the term "lipoprotein" means a biomolecular particle containing both protein and lipid. Examples of lipoproteins include, e.g., low density lipoprotein (LDL), high-density lipoprotein (HDL), very low density lipoprotein (VLDL), intermediate density lipoprotein (IDL), and lipoprotein (a) (Lp(a)).

The present invention, according to certain embodiments, relates to methods for treating patients who are non-responsive to, inadequately controlled by, or intolerant to standard lipid modifying therapy. As used herein, a particular patient who is "non-responsive to, inadequately controlled by, or intolerant to, standard lipid modifying therapy" is determined by a physician, physician's assistant, diagnostician, or other medical professional on the basis of the level of one or more lipoproteins (e.g., LDL-C and/or non-HDL-C) measured or otherwise detected in the serum of the patient after treatment with the standard lipid modifying agent. The physician, physician's assistant, diagnostician, or other medical professional can also determine if the patient is intolerant to standard lipid modifying therapies based on the side effect profile of the standard lipid modifying therapies, which the patient may experience, including, but not limited to, muscle aches, tenderness or weakness (myalgia), headache, skin flushing, difficulty sleeping, abdominal cramping, bloating, diarrhea, constipation, rash, nausea, or vomiting. A patient who is non-responsive to, inadequately controlled by, or intolerant to standard lipid modifying therapy may also be determined or influenced by other factors such as the patient's family history, medical background, current therapeutic treatment status, as well as generally accepted or prevailing lipoprotein targets adopted by national medical associations and physicians' groups. For example, in certain contexts, if a patient is undergoing therapy with a standard lipid modifying agent, and exhibits an LDL-C level of greater than or equal to about 70 mg/dL, this indicates that the patient is "non-responsive to, or inadequately controlled by, or intolerant to standard lipid modifying therapy" and may benefit by treatment using the therapies described herein. In other contexts, if a patient is undergoing therapy with a standard lipid modifying agent, and exhibits an LDL-C level of greater than or equal to about 100 mg/dL, this indicates that the patient is "non-responsive to, inadequately controlled by, or intolerant to standard lipid modifying therapy" and may benefit by treatment using the therapies described herein. In certain contexts, if a patient is undergoing therapy with a standard lipid modifying agent, and exhibits an LDL-C level of greater than or equal to about 150 mg/dL, 200 mg/dL, 250 mg/dL, 300 mg/dL, 400 mg/dL or higher, this indicates that the patient is "non-responsive to, inadequately controlled by, or intolerant to standard lipid modifying therapy" and may benefit by treatment using the therapies described herein. In yet other contexts, whether or not a particular percentage reduction in LDL-C or non-HDL-C level is met, relative to the patient's LDL-C or non-HDL-C level at a particular start point ("baseline") can be used to determine whether the patient has responded to standard lipid modifying therapy or whether that patient is in need of further treatment using the methods and agents of the present invention. For instance, a reduction in LDL-C or non-HDL-C of less than 50% (e.g., less than 40%, less than 35%, less than 30%, less than 25%, etc.) from baseline may signify a need for therapy using the methods and agents.

For example, in certain contexts, if a patient is undergoing therapy with a standard lipid modifying agent, and exhibits a triglyceride (TG) level of greater than or equal to about 150 mg/dL, this indicates that the patient is "non-responsive to, or inadequately controlled by, or intolerant to standard lipid modifying therapy" and may benefit by treatment using the therapies described herein. In other contexts, if a patient is undergoing therapy with a standard lipid modifying agent, and exhibits a TG level of greater than or equal to about 200 mg/dL, this indicates that the patient is "non-responsive to, inadequately controlled by, or intolerant to standard lipid modifying therapy" and may benefit by treatment using the therapies described herein. In certain contexts, if a patient is undergoing therapy with a standard lipid modifying agent, and exhibits a TG level of greater than or equal to about 300 mg/dL, 400 mg/dL, 500 mg/dL, 750 mg/dL, 1000 mg/dL or higher, this indicates that the patient is "non-responsive to, inadequately controlled by, or intolerant to standard lipid modifying therapy" and may benefit by treatment using the therapies described herein.

In yet other contexts, whether or not a particular percentage reduction in TG, TC, LDL-C or non-HDL-C level is met, relative to the patient's TG, TC, LDL-C or non-HDL-C level at a particular start point ("baseline") can be used to determine whether the patient has responded to standard lipid modifying therapy or whether that patient is in need of further treatment using the methods and agents of the present invention. For instance, a reduction in TG, TC, LDL-C or non-HDL-C of less than 50% (e.g., less than 40%, less than 35%, less than 30%, less than 25%, etc.) from baseline may signify a need for therapy using the methods and agents.

The present invention, accordingly, relates to methods of treatment comprising administration of one or more doses of an ANGPTL8 inhibitor combined with one or more doses of an ANGPTL3 inhibitor to a patient, whereby the patient's post-treatment levels of total cholesterol, TG, LDL-C, and/or non-HDL-C are significantly reduced in numbers. For example, the present invention relates to therapeutic methods comprising administering one or more doses of an ANGPTL8 inhibitor and one or more doses of an ANGPTL3 inhibitor to a patient who is undergoing standard lipid modifying therapy, but is non-responsive to such therapy, or is intolerant to such therapy, wherein, after receiving one or more doses of the ANGPTL8 inhibitor and one or more doses of the ANGPTL3 inhibitor, the patient is able to achieve normal levels of total cholesterol, TG, LDL-C, or non-LDL-C. In certain instances, the patient may be taken off of the standard lipid modifying therapy, or the standard lipid modifying therapy may be continued, but may be administered at lower doses and may be used in combination with the ANGPTL8 inhibitor and the ANGPTL3 inhibitor, to achieve and/or maintain a particular target lipoprotein level. Alternatively, the patient may be administered the standard lipid modifying therapy at the normal prescribed dose, but the frequency of administration of the lipid modifying therapy may be reduced if the standard lipid modifying therapy is to be administered in conjunction with the combination of the ANGPTL8 inhibitor and the ANGPTL3 inhibitor. In some instances, the need for treatment with the standard lipid modifying therapy by the patient to achieve and/or maintain a particular target lipoprotein level may be eliminated altogether following administration of one or more doses of the ANGPTL8 inhibitor in conjunction with the ANGPTL3 inhibitor.

According to certain embodiments, the present invention relates to methods for reducing or eliminating the need for standard lipid modifying therapy, wherein the methods comprise selecting a patient with hyperlipidemia (*e*.*g*., hypercholesterolemia, or hypertriglyceridemia) who has been treated with lipid modifying therapy within the last month, the last 2 months, the last 3 months, the last 4 months, the last 5 months, the last 6 months, or for a longer period, and administering one or more doses of a ANGPTL8 inhibitor in combination with an ANGPTL3 inhibitor to the patient. The methods according to this aspect result in lowering the level of at least one lipoprotein in the serum of the patient, and consequently allow for a reduction or elimination of the need for treatment with the standard lipid modifying therapy by the patient. For example, in certain embodiments of the present invention, following administration of one or more doses of a ANGPTL8 inhibitor in combination with an ANGPTL3 inhibitor, the serum LDL-C level of the patient is reduced to less than a defined level (*e*.*g*., less than 100 mg/dL or less than 70 mg/dL), or the total cholesterol is lowered to a defined level (*e*.*g*. less than 200 mg/dL, or less than 150 mg/dL), or the TG level is reduced to a defined level (*e*.*g*. less than 200 mg/dL, or less than 150 mg/dL).

According to certain embodiments, the patient who is treatable by the methods has hypercholesterolemia (*e*.*g*., a serum LDL-C concentration of greater than or equal to 70 mg/dL, or a serum LDL-C concentration greater than or equal to 100 mg/dL). According to certain embodiments, the patient who is treatable by the methods of the present invention has hypertriglyceridemia (*e*.*g*., a serum TG concentration of greater than or equal to 150 mg/dL, or a serum TG concentration greater than or equal to 200 mg/dL). In certain embodiments, the patient's hypercholesterolemia is inadequately controlled by standard lipid modifying therapy, e.g. statin therapy. For example, the present invention relates to methods for treating a patient who is non-responsive, inadequately controlled by, or intolerant to, therapy with a standard lipid modifying therapy, such as a statin, or who has hypercholesterolemia that is inadequately controlled by a daily dose of a statin selected form the group consisting of atorvastatin (including atorvastatin + ezetimibe), rosuvastatin, cerivastatin, pitavastatin, fluvastatin, lovastatin, simvastatin (including simvastatin + ezetimibe), pravastatin, and combinations thereof. The present invention also relates to methods for reducing cholesterol, TG, LDL-C, or non-LDL-C in a patient who has hypercholesterolemia, or hypertriglyceridemia and who exhibits statin intolerance or who otherwise experiences adverse or undesirable reaction(s) to statin therapy (*e*.*g*., skeletal muscle pain, aches, weakness or cramping [e.g., myalgia, myopathy, rhabdomyolysis, etc.]).

### Patient Selection

The present invention relates to methods and compositions useful for treating patients who are suffering from hyperlipidemia, who are non-responsive to, inadequately controlled by, or intolerant to, therapy with a standard lipid modifying therapy. The patients who are treatable by the methods may also exhibit one or more of additional selection criteria. For example, a patient may be selected for treatment if the patient is diagnosed with or identified as being at risk of developing a hypercholesterolemia condition such as, *e*.*g*., heterozygous Familial Hypercholesterolemia (heFH), homozygous Familial Hypercholesterolemia (hoFH), Autosomal Dominant Hypercholesterolemia, autosomal recessive hypercholesterolemia (ARH, *e*.*g*., ARH associated with mutations in LDLRAP1), as well as incidences of hypercholesterolemia that are distinct from Familial Hypercholesterolemia (nonFH). Diagnosis of familial hypercholesterolemia (*e*.*g*., heFH or hoFH) can be made by genotyping and/or clinical criteria. For patients who are not genotyped, clinical diagnosis may be based on either the Simon Broome criteria with a criteria for definite FH, or the WHO/Dutch Lipid Network criteria with a score > 8 points.

According to certain embodiments, the patient may be selected on the basis of having a history of coronary heart disease (CHD). As used herein a "history of CHD" (or "documented history of CHD") includes one or more of: (i) acute myocardial infarction (MI); (ii) silent MI; (iii) unstable angina; (iv) coronary revascularization procedure (e.g., percutaneous coronary intervention [PCI] or coronary artery bypass graft surgery [CABG]); and/or (v) clinically significant CHD diagnosed by invasive or non-invasive testing (such as coronary angiography, stress test using treadmill, stress echocardiography or nuclear imaging).

According to certain embodiments, the patient may be selected on the basis of having non-coronary heart disease cardiovascular disease ("non-CHD CVD"). As used herein, "non-CHD CVD" includes one or more of: (i) documented previous ischemic stroke with a focal ischemic neurological deficit that persisted more than 24 hours, considered as being of atherothrombotic origin; (ii) peripheral arterial disease; (iii) abdominal aortic aneurysm; (iv) atherosclerotic renal artery stenosis; and/or (v) carotid artery disease (transient ischemic attacks or >50% obstruction of a carotid artery).

According to certain embodiments, the patient may be selected on the basis of having one or more additional risk factors such as, *e*.*g*., (i) documented moderate chronic kidney disease (CKD) as defined by 30 ≤eGFR <60 mL/min/1.73 m2 for 3 months or more; (ii) type 1 or type 2 diabetes mellitus with or without target organ damage (*e*.*g*., retinopathy, nephropathy, microalbuminuria); (iii) a calculated 10-year fatal CVD risk SCORE ≥5% (ESC/EAS Guidelines for the management of dyslipidemias, Conroy et al., 2003, Eur. Heart J. 24:987-1003).

According to certain embodiments, the patient may be selected on the basis of having one or more additional risk factors selected from the group consisting of age (*e*.*g*., older than 40, 45, 50, 55, 60, 65, 70, 75, or 80 years), race, national origin, gender (male or female), exercise habits (*e*.*g*., regular exerciser, non-exerciser), other preexisting medical conditions *(e.g.,* type-II diabetes, high blood pressure, etc.), and current medication status (*e.g.,* currently taking beta blockers, niacin, ezetimibe, fibrates, omega-3 fatty acids, bile acid resins, etc.).

According to certain embodiments of the present invention, the subject who is treatable by the methods exhibits an elevated level of one or more inflammatory marker. Any marker of systemic inflammation can be utilized for the purposes of the present invention. Suitable inflammatory markers include, without limitation, C-reactive protein, cytokines (*e*.*g*., II-6, IL-8, and/or IL-17), and cellular adhesion molecules (*e*.*g*., ICAM-1, ICAM-3, BL-CAM, LFA-2, VCAM-1, NCAM, and PECAM).

According to the present invention, patients may be selected on the basis of a combination of one or more of the foregoing selection criteria or therapeutic characteristics. For example, according to certain embodiments, a patient suitable for treatment with the methods, may further be selected on the basis of having heFH or non-FH in combination with: (i) a history of documented CHD, (ii) non-CHD CVD, and/or (iii) diabetes mellitus with target organ damage; such patients may also be selected on the basis of having a serum LDL-C concentration of greater than or equal to 70 mg/dL.

According to certain other embodiments, a patient suitable for treatment, in addition to having hypercholesterolemia that is not adequately controlled by a daily moderate-dose therapeutic statin regimen, may further be selected on the basis of having heFH or non-FH without CHD, or non-CHD CVD, but having either (i) a calculated 10-year fatal CVD risk SCORE ≥5%; or (ii) diabetes mellitus without target organ damage; such patients may also be selected on the basis of having a serum LDL-C concentration of greater than or equal to 100 mg/dL.

According to certain embodiments of the present invention, the subject who is treatable is a subject who has familial chylomicronemia syndrome (FCS; also known as lipoprotein lipase deficiency).

According to certain embodiments of the present invention, the subject who is treatable is a subject who is undergoing, or has recently undergone, lipoprotein apheresis (*e*.*g*., within the last six months, within the last 12 weeks, within the last 8 weeks, within the last 6 weeks, within the last 4 weeks, within the last 2 weeks, etc.).

### Administration of an ANGPTL8 Inhibitor plus an ANGPTL3 Inhibitor as Add-On Therapy

The present invention relates to methods of treatment wherein a patient who is undergoing, or has recently undergone, standard lipid modifying therapy (*e*.*g*. a statin) is administered an ANGPTL8 inhibitor plus an ANGPTL3 inhibitor according to a particular dosing amount and frequency, and wherein the ANGPTL8 inhibitor and the ANGPTL3 inhibitor are administered as an add-on to the patient's pre-existing lipid modifying therapy (if applicable), such as an add-on to the patient's pre-existing daily therapeutic statin regimen.

For example, the methods include add-on therapeutic regimens wherein the ANGPTL8 inhibitor and the ANGPTL3 inhibitor are administered as add-on therapy to the same stable daily therapeutic statin regimen (*i.e.,* same dosing amount of statin) that the patient was on prior to receiving the ANGPTL8 and ANGPTL3 inhibitors. In other embodiments, the ANGPTL8 and ANGPTL3 inhibitors are administered as add-on therapy to a therapeutic statin regimen comprising a statin in an amount that is more than or less than the dose of statin the patient was on prior to receiving the ANGPTL8 and ANGPTL3 inhibitors. For example, after starting a therapeutic regimen comprising a ANGPTL8 inhibitor and an ANGPTL3 inhibitor administered at particular dosing frequencies and amounts, the daily dose of statin administered or prescribed to the patient may (a) stay the same, (b) increase, or (c) decrease (*e*.*g*., up-titrate or down-titrate) in comparison to the daily statin dose the patient was taking before starting the ANGPTL8 and ANGPTL3 inhibitors therapeutic regimen, depending on the therapeutic needs of the patient.

### Therapeutic Efficacy

The methods may result in the reduction in serum levels of one or more lipid components selected from the group consisting of total cholesterol, LDL-C, non-HDL-C, ApoB100, VLDL-C, triglyceride (TG), Lp(a) and remnant cholesterol. For example, according to certain embodiments of the present invention, administration of an ANGPTL8 inhibitor in combination with an ANGPTL3 inhibitor to a suitable subject will result in a mean percent reduction from baseline in serum low density lipoprotein cholesterol (LDL-C) of at least about 25%, 30%, 40%, 50%, 60%, or greater; a mean percent reduction from baseline in ApoB100 of at least about 25%, 30%, 40%, 50%, 60%, or greater; a mean percent reduction from baseline in non-HDL-C of at least about 25%, 30%, 40%, 50%, 60%, or greater; a mean percent reduction from baseline in total cholesterol of at least about 10%, 15%, 20%, 25%, 30%, 35%, or greater; a mean percent reduction from baseline in VLDL-C of at least about 5%, 10%, 15%, 20%, 25%, 30%, or greater; a mean percent reduction from baseline in triglycerides of at least about 5%, 10%, 15%, 20%, 25%, 30%, 35% or greater; and/or a mean percent reduction from baseline in Lp(a) of at least about 5%, 10%, 15%, 20%, 25%, or greater.

### ANGPTL8 Inhibitors and ANGPTL3 Inhibitors

The methods comprise administering to a patient a therapeutic composition comprising an ANGPTL8 inhibitor and an ANGPTL3 inhibitor.

### ANGPTL8 Inhibitors

As used herein, a "ANGPTL8 inhibitor" is any agent, which binds to or interacts with human ANGPTL8 and inhibits the normal biological function of ANGPTL8 *in vitro* or *in vivo.* Non-limiting examples of categories of ANGPTL8 inhibitors include small molecule ANGPTL8 antagonists, nucleic acid-based inhibitors of ANGPTL8 expression or activity (e.g., siRNA or antisense), peptide-based molecules that specifically interact with ANGPTL8 (e.g., peptibodies), receptor molecules that specifically interact with ANGPTL8, proteins comprising a ligand-binding portion of an LDL receptor, ANGPTL8-binding scaffold molecules (e.g., DARPins, HEAT repeat proteins, ARM repeat proteins, tetratricopeptide repeat proteins, fibronectin-based scaffold constructs, and other scaffolds based on naturally occurring repeat proteins, etc., [*see, e.g.,* Boersma and Pluckthun, 2011, Curr. Opin. Biotechnol. 22:849-857, and references cited therein]), and anti-ANGPTL8 aptamers or portions thereof. In the present invention, the ANGPTL8 inhibitor is an anti-ANGPTL8 antibody or antigen-binding fragment of an antibody that specifically bind human ANGPTL8 as defined in the claims.

The term "human angiopoietin-like protein 8" or "human ANGPTL8" or "hANGPTL8", or "ANGPTL8", is also referred to as "lipasin", "RIFL", or "betatrophin". Human ANGPTL8 also refers to ANGPTL8 having the protein sequence shown in amino acids 1-177 of SEQ ID NO: 9, or a biologically active fragment thereof, as well as that shown in GenBank accession number NP_061157.3 (SEQ ID NO:19)). The activity of ANGPTL8 that can be neutralized, inhibited, blocked, abrogated, reduced or interfered with, by an antibody or antigen-binding fragment thereof, includes, but is not limited to, inhibition of LPL activity, or lowering of triglyceride levels *in vivo* and the like.

### ANGPTL3 Inhibitors

As used herein, an "ANGPTL3 inhibitor" is any agent, which binds to or interacts with human ANGPTL3 and inhibits the normal biological function of ANGPTL3 *in vitro* or *in vivo.* Non-limiting examples of categories of ANGPTL3 inhibitors include small molecule ANGPTL3 antagonists, nucleic acid-based inhibitors of ANGPTL3 expression or activity (e.g., siRNA or antisense), peptide-based molecules that specifically interact with ANGPTL3 (e.g., peptibodies), receptor molecules that specifically interact with ANGPTL3, ANGPTL3-binding scaffold molecules (e.g., DARPins, HEAT repeat proteins, ARM repeat proteins, tetratricopeptide repeat proteins, fibronectin-based scaffold constructs, and other scaffolds based on naturally occurring repeat proteins, etc., [*see, e.g.,* Boersma and Pluckthun, 2011, Curr. Opin. Biotechnol. 22:849-857, and references cited therein]), and anti-ANGPTL3 aptamers or portions thereof. In the invention, ANGPTL3 inhibitors are anti-ANGPTL3 antibodies or antigen-binding fragments of antibodies that specifically bind human ANGPTL3 as defined in the claims.

The term "human angiopoietin-like protein-3" or "human ANGPTL3" or "hANGPTL3", as used herein, refers to ANGPTL3 having the amino acid sequence of SEQ ID NO: 18 (see also NCBI Accession NP_055310), or a biologically active fragment thereof.

The term "antibody", as used herein, is intended to refer to immunoglobulin molecules comprising four polypeptide chains, two heavy (H) chains and two light (L) chains interconnected by disulfide bonds, as well as multimers thereof (e.g., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments of the invention, the FRs of the anti-ANGPTL8 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e*.*g*., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (*e*.*g*. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR, which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

An antigen-binding fragment of an antibody may contain at least one variable domain covalently linked to at least one constant domain. Non-limiting, exemplary configurations of variable and constant domains that may be found within an antigen-binding fragment of an antibody include: (i) V_{H}-C_{H}1; (ii) V_{H}-C_{H}2; (iii) V_{H}-C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e*.*g*., 5, 10, 15, 20, 40, 60 or more) amino acids, which result in a flexible or semi-flexible linkage between adjacent variable and/or constant domains in a single polypeptide molecule. Moreover, an antigen-binding fragment of an antibody may comprise a homo-dimer or hetero-dimer (or other multimer) of any of the variable and constant domain configurations listed above in non-covalent association with one another and/or with one or more monomeric V_{H} or V_{L} domain (*e*.*g*., by disulfide bond(s)).

As with full antibody molecules, antigen-binding fragments may be monospecific or multispecific (*e*.*g*., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies may nonetheless include amino acid residues not encoded by human germline immunoglobulin sequences (e.g., mutations introduced by random or site-specific mutagenesis *in vitro* or by somatic mutation *in vivo*)*,* for example in the CDRs and in particular CDR3. However, the term "human antibody", as used herein, is not intended to include antibodies in which CDR sequences derived from the germline of another mammalian species, such as a mouse, have been grafted onto human framework sequences. The term includes antibodies recombinantly produced in a non-human mammal, or in cells of a non-human mammal. The term is not intended to include antibodies isolated from or generated in a human subject.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

Human antibodies can exist in two forms that are associated with hinge heterogeneity. In one form, an immunoglobulin molecule comprises a stable four-chain construct of approximately 150-160 kDa in which the dimers are held together by an inter-chain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant invention encompasses antibodies having one or more mutations in the hinge, C_{H}2 or C_{H}3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" ANGPTL8, or that "specifically binds" ANGPTL3, as used in the context of the present invention, includes antibodies that bind ANGPTL8, or ANGPTL3, or a portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human ANGPTL8, or human ANGPTL3, however, has cross-reactivity to other antigens, such as ANGPTL8 molecules, or ANGPTL3 molecules from other (non-human) species.

The anti-ANGPTL8 and the anti-ANGPTL3 antibodies useful for the methods of the present disclosure may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present disclosure includes methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain aspects of the disclosure, all of the framework and/or CDR residues within the V_{H} and/or V_{L} domains are mutated back to the residues found in the original germline sequence from which the antibody was derived. In other aspects, only certain residues are mutated back to the original germline sequence, *e*.*g*., only the mutated residues found within the first 8 amino acids of FR1 or within the last 8 amino acids of FR4, or only the mutated residues found within CDR1, CDR2 or CDR3. In other aspects, one or more of the framework and/or CDR residue(s) are mutated to the corresponding residue(s) of a different germline sequence (*i.e.,* a germline sequence that is different from the germline sequence from which the antibody was originally derived). Furthermore, the antibodies of the present disclosure may contain any combination of two or more germline mutations within the framework and/or CDR regions, *e*.*g*., wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc.

The present disclosure also includes methods involving the use of anti-ANGPTL8, and anti-ANGPTL3 antibodies comprising variants of any of the HCVR, LCVR, and/or CDR amino acid sequences disclosed herein having one or more conservative substitutions. For example, the present disclosure includes the use of anti-ANGPTL8, and anti-ANGPTL3 antibodies having HCVR, LCVR, and/or CDR amino acid sequences with, *e*.*g*., 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance", as used herein, refers to an optical phenomenon that allows for the analysis of real-time interactions by detection of alterations in protein concentrations within a biosensor matrix, for example using the BIAcore™ system (Biacore Life Sciences division of GE Healthcare, Piscataway, NJ).

The term "K_{D}", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

The anti-ANGPTL8 and anti-ANGPTL3 antibodies used in the methods may be antibodies with pH-dependent binding characteristics. As used herein, the expression "pH-dependent binding" means that the antibody or antigen-binding fragment thereof exhibits "reduced binding to ANGPTL8 at acidic pH as compared to neutral pH" (for purposes of the present disclosure, both expressions may be used interchangeably), or that the antibody or antigen-binding fragment thereof exhibits "reduced binding to ANGPTL3 at acidic pH as compared to neutral pH" (for purposes of the present disclosure, both expressions may be used interchangeably). For the example, antibodies "with pH-dependent binding characteristics" includes antibodies and antigen-binding fragments thereof that bind either to ANGPTL8, or to ANGPTL3 with higher affinity at neutral pH than at acidic pH. In certain aspects, the antibodies and antigen-binding fragments bind ANGPTL8, or ANGPTL3 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

The anti-ANGPTL8 antibodies, or the ANGPTL3 antibodies with pH-dependent binding characteristics may possess one or more amino acid variations relative to the parental anti-ANGPTL8 antibody, or the parental anti-ANGPTL3 antibody. For example, an anti-ANGPTL8 antibody, or an anti-ANGPTL3 antibody with pH-dependent binding characteristics may contain one or more histidine substitutions or insertions, e.g., in one or more CDRs of a parental anti-ANGPTL8, or a parental anti-ANGPTL3 antibody. Thus, according to certain aspects of the disclosure, methods are provided comprising administering an anti-ANGPTL8 antibody and an anti-ANGPTL3 antibody which comprises CDR amino acid sequences (*e*.*g*., heavy and light chain CDRs) which are identical to the CDR amino acid sequences of a parental anti-ANGPTL8 antibody, or parental ANGPTL3 antibody except for the substitution of one or more amino acids of one or more CDRs of the parental antibody with a histidine residue. The anti-ANGPTL8 antibodies, or anti-ANGPTL3 antibodies with pH-dependent binding may possess, *e*.*g*., 1, 2, 3, 4, 5, 6, 7, 8, 9, or more histidine substitutions, either within a single CDR of a parental antibody or distributed throughout multiple (*e*.*g*., 2, 3, 4, 5, or 6) CDRs of a parental anti-ANGPTL8 antibody, or a parental anti-ANGPTL3 antibody. For example, the present disclosure includes the use of anti-ANGPTL8 antibodies and anti-ANGPTL3 antibodies with pH-dependent binding comprising one or more histidine substitutions in HCDR1, one or more histidine substitutions in HCDR2, one or more histidine substitutions in HCDR3, one or more histidine substitutions in LCDR1, one or more histidine substitutions in LCDR2, and/or one or more histidine substitutions in LCDR3, of a parental anti-ANGPTL8 antibody, or a parental anti-ANGPTL3 antibody.

As used herein, the expression "acidic pH" means a pH of 6.0 or less (*e*.*g*., less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

The anti-ANGPTL8 antibody used in the context of the present disclosure includes an anti-ANGPTL8 antibody comprising the three heavy chain complementarity determining regions (HCDRs) contained in the heavy chain variable region (HCVR) of SEQ ID NO: 1 and the three light chain complementarity determining regions (LCDRs) contained in the light chain variable region (LCVR) of SEQ ID NO: 5. In the present invention, the anti-ANGPTL8 antibody comprises the HCDR1 of SEQ ID NO: 2, the HCDR2 of SEQ ID NO: 3, the HCDR3 of SEQ ID NO: 4, the LCDR1 of SEQ ID NO: 6, the LCDR2 of SEQ ID NO: 7, and the LCDR3 of SEQ ID NO: 8. In one embodiment, the anti-ANGPTL8 antibody that can be used in the context of the present invention includes an anti-ANGPTL8 antibody comprising the HCVR/LCVR amino acid sequence pair of SEQ ID NOs: 1/5.

The anti-ANGPTL3 antibody used in the context of the present invention includes evinacumab, which comprises the three heavy chain complementarity determining regions (HCDRs) contained in the heavy chain variable region (HCVR) of SEQ ID NO: 10 and the three light chain complementarity determining regions (LCDRs) contained in the light chain variable region (LCVR) of SEQ ID NO: 14. Evinacumab comprises the HCDR1 of SEQ ID NO: 11, the HCDR2 of SEQ ID NO: 12, the HCDR3 of SEQ ID NO: 13, the LCDR1 of SEQ ID NO: 15, the LCDR2 of SEQ ID NO: 16, and the LCDR3 of SEQ ID NO: 17. Evinacumab comprises the HCVR/LCVR amino acid sequence pair of SEQ ID NOs: 10/14.

### Preparation of Human Antibodies

Anti-ANGPTL8 antibodies and anti-ANGPTL3 antibodies can be made according to any method of antibody production/isolation known in the art. For example, antibodies for use in the methods of the present invention may be made by hybridoma technologies, by phage display, by yeast display, etc. Antibodies for use in the methods of the present invention may be, *e*.*g*., chimeric antibodies, humanized antibodies, or fully human antibodies.

Methods for generating human antibodies in transgenic mice are known in the art. Any such known methods can be used in the context of the present invention to make human antibodies that specifically bind ANGPTL8, or ANGPTL3.

For example, using VELOCIMMUNE™ technology (see, for example, US 6,596,541, Regeneron Pharmaceuticals) or any other known method for generating monoclonal antibodies, high affinity chimeric antibodies to ANGPTL8, or to ANGPTL3 are initially isolated having a human variable region and a mouse constant region. The VELOCIMMUNE® technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE® mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc., using standard procedures known to those skilled in the art. The mouse constant regions are replaced with a desired human constant region to generate the fully human antibody of the invention, for example wild-type or modified IgG1 or IgG4. While the constant region selected may vary according to specific use, high affinity antigen-binding and target specificity characteristics reside in the variable region.

In general, the antibodies that can be used in the methods of the present invention possess high affinities, as described above, when measured by binding to antigen either immobilized on solid phase or in solution phase. The mouse constant regions are replaced with desired human constant regions to generate the fully human antibodies of the invention. While the constant region selected may vary according to specific use, high affinity antigen-binding and target specificity characteristics reside in the variable region.

Specific examples of human antibodies or antigen-binding fragments of antibodies that specifically bind ANGPTL8, which can be used in the context of the methods of the present disclosure include antibodies or antigen-binding proteins comprising the six CDRs (HCDR1, HCDR2, HCDR3, LCDR1, LCDR2 and LCDR3) from the heavy and light chain variable region (HCVR/LCVR) amino acid sequence pair comprising SEQ ID NOs: 1/5.

In the present invention, the anti-ANGPTL8 antibody, or antigen-binding fragment thereof, comprises heavy and light chain complementarity determining regions (HCDR1-HCDR2-HCDR3/LCDR1-LCDR2-LCDR3) comprising the amino acid sequences of SEQ ID NOs: 2, 3, 4, 6, 7 and 8.

In certain embodiments of the present invention, the anti-ANGPTL8 antibody, or antigen-binding fragment thereof, that can be used in the methods comprises an HCVR having the amino acid sequence of SEQ ID NO:1 and an LCVR having the amino acid sequence of SEQ ID NO:5.

Specific examples of human antibodies or antigen-binding fragments of antibodies that specifically bind ANGPTL3, which can be used in the context of the methods of the present disclosure include antibodies or antigen-binding proteins comprising the six CDRs (HCDR1, HCDR2, HCDR3, LCDR1, LCDR2 and LCDR3) from the heavy and light chain variable region (HCVR/LCVR) amino acid sequence pair comprising SEQ ID NOs: 10/14.

In the present invention, the anti-ANGPTL3 antibody, or antigen-binding fragment thereof, comprises heavy and light chain complementarity determining regions (HCDR1-HCDR2-HCDR3/LCDR1-LCDR2-LCDR3) comprising the amino acid sequences of SEQ ID NOs:11, 12, 13, 15, 16 and 17.

In certain embodiments of the present invention, the anti-ANGPTL3 antibody, or antigen-binding fragment thereof, that can be used in the methods comprises an HCVR having the amino acid sequence of SEQ ID NO:10 and an LCVR having the amino acid sequence of SEQ ID NO:14.

### Pharmaceutical Compositions and Methods of Administration

The present invention relates to methods, which comprise administering an ANGPTL8 inhibitor to a patient in combination with an ANGPTL3 inhibitor, wherein the ANGPTL8 inhibitor and the ANGPTL3 inhibitor are contained within the same, or in different pharmaceutical compositions. The pharmaceutical compositions may be formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Exemplary pharmaceutical formulations comprising anti-ANGPTL8 antibodies, and/or ANGPTL3 antibodies that can be used in the context of the present invention include any of the formulations as set forth in US 8,795,669, or in WO2013/166448, or WO2012/168491.

Various delivery systems are known and can be used to administer the pharmaceutical composition, *e*.*g*., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition can be delivered subcutaneously or intravenously with a standard needle and syringe. In addition, with respect to subcutaneous delivery, a pen delivery device readily has applications in delivering a pharmaceutical composition. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition. Examples include, but are not limited to AUTOPEN™ (Owen Mumford, Inc., Woodstock, UK), DlSETRONlC™ pen (Disetronic Medical Systems, Bergdorf, Switzerland), HUMALOG MIX 75/25™ pen, HUMALOG™ pen, HUMALIN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™, and OPTICLIK™ (Sanofi-Aventis, Frankfurt, Germany), to name only a few. Examples of disposable pen delivery devices having applications in subcutaneous delivery of a pharmaceutical composition of the present invention include, but are not limited to the SOLOSTAR™ pen (Sanofi-Aventis), the FLEXPEN™ (Novo Nordisk), and the KWIKPEN™ (Eli Lilly), the SURECLICK™ Autoinjector (Amgen, Thousand Oaks, CA), the PENLET™ (Haselmeier, Stuttgart, Germany), the EPIPEN (Dey, L.P.), and the HUMIRA™ Pen (Abbott Labs, Abbott Park IL), to name only a few.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, *e*.*g*., Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

### Dosage

The amount of an ANGPTL8 inhibitor (*e*.*g*., anti-ANGPTL8 antibody), or an ANGPTL3 inhibitor (*e*.*g*., anti-ANGPTL3 antibody) administered to a subject is, generally, a therapeutically effective amount. As used herein, the phrase "therapeutically effective amount of a ANGPTL8 inhibitor" means a dose of a ANGPTL8 inhibitor, when administered in combination with an ANGPTL3 inhibitor, results in a detectable reduction (at least about 5%, 10%, 15%, 20%, 25%, 30%, 35%, 40%, 45%, 50%, 55%, 60%, 65%, 70%, 75%, or more from baseline) in one or more parameters selected from the group consisting of total cholesterol, LDL-C, ApoB100, non-HDL-C, VLDL-C, triglycerides, Lp(a) and remnant cholesterol, or an amount that reduces or eliminates a patient's need for other therapeutic interventions, such as, lipoprotein apheresis, or that reduces a patient's normalized rate of apheresis.

In the case of an anti-ANGPTL8 antibody, a therapeutically effective amount can be from about 0.05 mg to about 600 mg, e.g., about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-ANGPTL8 antibody. According to certain exemplary embodiments of the present invention, a therapeutically effective amount of an anti-ANGPTL8 antibody is 75 mg, 150 mg or 300 mg (*e.g.,* in the case of alirocumab), or 140 mg or 420 mg (*e.g.,* in the case of evolocumab). Other dosing amounts of ANGPTL8 inhibitors will be apparent to persons of ordinary skill in the art.

The amount of anti-ANGPTL8 antibody contained within the individual doses may be expressed in terms of milligrams of antibody per kilogram of patient body weight (*i.e.,* mg/kg). For example, the anti-ANGPTL8 antibody may be administered to a patient at a dose of about 0.0001 to about 10 mg/kg of patient body weight.

The amount of ANGPTL3 inhibitor (*e*.*g*., anti-ANGPTL3 antibody) administered to a subject is, generally, a therapeutically effective amount. As used herein, the phrase "therapeutically effective amount of an ANGPTL3 inhibitor" means a dose of ANGPTL3 inhibitor, when combined with a ANGPTL8 inhibitor, results in a detectable reduction (at least about 5%, 10%, 15%, 20%, 25%, 30%, 35%, 40%, 45%, 50%, 55%, 60%, 65%, 70%, 75%, or more from baseline) in one or more parameters selected from the group consisting of total cholesterol, LDL-C, ApoB100, non-HDL-C, VLDL-C, triglycerides, Lp(a) and remnant cholesterol, or an amount that prevents or attenuates atherosclerosis in a subject (as described elsewhere herein).

In the case of an anti-ANGPTL3 antibody, a therapeutically effective amount can be from about 0.05 mg to about 600 mg, *e*.*g*., about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-ANGPTL3 antibody. Other dosing amounts of ANGPTL3 inhibitors will be apparent to persons of ordinary skill in the art.

The amount of anti-ANGPTL3 antibody contained within the individual doses may be expressed in terms of milligrams of antibody per kilogram of patient body weight (*i.e.,* mg/kg). For example, the anti-ANGPTL3 antibody may be administered to a patient at a dose of about 0.0001 to about 10 mg/kg of patient body weight.

### Combination Therapies

As described elsewhere herein, the methods may comprise administering an ANGPTL8 inhibitor in combination with an ANGPTL3 inhibitor to a patient who is non-responsive to, inadequately controlled by, or intolerant to a standard lipid lowering therapy. In certain embodiments, the need for further administration of the lipid lowering therapy may be eliminated altogether. In certain embodiments, the combined use of the ANGPTL8 inhibitor with the ANGPTL3 inhibitor may be used in combination with ("on top of") the patient's previously prescribed lipid lowering therapy. For example, in the context of lowering at least one lipid/lipoprotein parameter in a patient suffering from hyperlipidemia (*e*.*g*. hypercholesterolemia, or hypertriglyceridemia), wherein the patient is non-responsive to, inadequately controlled by, or intolerant to a standard lipid lowering therapy, a combination of a ANGPTL8 inhibitor with an ANGPTL3 inhibitor may be administered to a patient in combination with a stable daily therapeutic statin regimen. Exemplary daily therapeutic statin regimens that a ANGPTL8 inhibitor plus an ANGPTL3 inhibitor may be administered in combination with in the context of the present invention include, *e*.*g*., atorvastatin (10, 20, 40 or 80 mg daily), (atorvastatin/ezetimibe 10/10 or 40/10 mg daily), rosuvastatin (5, 10 or 20 mg daily), cerivastatin (0.4 or 0.8 mg daily), pitavastatin (1, 2 or 4 mg daily), fluvastatin (20, 40 or 80 mg daily), simvastatin (5, 10, 20, 40 or 80 mg daily), simvastatin/ezetimibe (10/10, 20/10, 40/10 or 80/10 mg daily), lovastatin (10, 20, 40 or 80 mg daily), pravastatin (10, 20, 40 or 80 mg daily), and combinations thereof. Other lipid modifying therapies that a ANGPTL8 inhibitor plus an ANGPTL3 inhibitor may be administered in combination with in the context of the present invention include, *e*.*g*., (1) an agent which inhibits cholesterol uptake and or bile acid reabsorption (*e*.*g*., ezetimibe); (2) an agent which increase lipoprotein catabolism (such as niacin); and/or (3) activators of the LXR transcription factor that plays a role in cholesterol elimination such as 22-hydroxycholesterol.

A non-limiting example of an anti-ANGPTL8 antibody that can be used in the context of the present invention includes an antibody comprising the HCVR/LCVR amino acid sequence pair of SEQ ID NOs: 1/5, or an antigen-binding portion thereof.

A non-limiting example of an ANGPTL3 antibody to be used in the context of the present invention includes evinacumab, comprising the HCVR/LCVR amino acid sequence pair of SEQ ID NOs: 10/14, or an antigen-binding portion thereof.

### Administration Regimens

According to certain embodiments of the present invention, multiple doses of an ANGPTL8 inhibitor (*i.e.,* a pharmaceutical composition comprising an ANGPTL8 inhibitor) and an ANGPTL3 inhibitor (*i.e.,* a pharmaceutical composition comprising an ANGPTL3 inhibitor) may be administered to a subject over a defined time course (*e*.*g*., on top of a daily therapeutic statin regimen or other background lipid modifying therapy). The methods according to this aspect of the invention comprise sequentially administering to a subject multiple doses of an ANGPTL8 inhibitor and an ANGPTL3 inhibitor. As used herein, "sequentially administering" means that each dose of ANGPTL8 inhibitor and ANGPTL3 inhibitor is administered to the subject at a different point in time, *e*.*g*., on different days separated by a predetermined interval (*e*.*g*., hours, days, weeks or months). The present invention includes sequentially administering to the patient a single initial dose of an ANGPTL8 inhibitor and an ANGPTL3 inhibitor, followed by one or more secondary doses of the ANGPTL8 inhibitor and ANGPTL3 inhibitor, and optionally followed by one or more tertiary doses of the ANGPTL8 inhibitor and ANGPTL3 inhibitor.

The terms "initial dose," "secondary doses," and "tertiary doses," refer to the temporal sequence of administration of the individual doses of a pharmaceutical composition comprising an ANGPTL8 inhibitor and the ANGPTL3 inhibitor. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of the ANGPTL8 inhibitor and the ANGPTL3 inhibitor, but generally may differ from one another in terms of frequency of administration. In certain embodiments, however, the amount of ANGPTL8 inhibitor and the ANGPTL3 inhibitor contained in the initial, secondary and/or tertiary doses varies from one another (*e*.*g*., adjusted up or down as appropriate) during the course of treatment. In certain embodiments, two or more (*e*.*g*., 2, 3, 4, or 5) doses are administered at the beginning of the treatment regimen as "loading doses" followed by subsequent doses that are administered on a less frequent basis (*e*.*g*., "maintenance doses").

According to exemplary embodiments of the present invention, each secondary and/or tertiary dose is administered 1 to 26 (e.g., 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, 15, 15½, 16, 16½, 17, 17½, 18, 18½, 19, 19½, 20, 20½, 21, 21½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of antigen-binding molecule, which is administered to a patient prior to the administration of the very next dose in the sequence with no intervening doses.

The methods according to this aspect of the invention may comprise administering to a patient any number of secondary and/or tertiary doses of an ANGPTL8 inhibitor and ANGPTL3 inhibitor. For example, in certain embodiments, only a single secondary dose is administered to the patient. In other embodiments, two or more (e.g., 2, 3, 4, 5, 6, 7, 8, or more) secondary doses are administered to the patient. Likewise, in certain embodiments, only a single tertiary dose is administered to the patient. In other embodiments, two or more (*e*.*g*., 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 2, 4, 6, 8 or more weeks after the immediately preceding dose. Similarly, in embodiments involving multiple tertiary doses, each tertiary dose may be administered at the same frequency as the other tertiary doses. For example, each tertiary dose may be administered to the patient 1 to 2, 4, 6, 8 or more weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human ANGPTL8

Anti-ANGPTL8 antibodies were obtained by immunizing a VELOCIMMUNE® mouse (*i.e.,* an engineered mouse comprising DNA encoding human immunoglobulin heavy and kappa light chain variable regions) with an immunogen comprising a recombinant human ANGPTL8 expressed with a C-terminal mouse IgG2a Fc tag (See SEQ ID NO: 9). The antibody immune response was monitored by an ANGPTL8-specific immunoassay. When a desired immune response was achieved, several fully human anti-ANGPTL8 antibodies were generated from antigen-positive B cells as described in US 2007/0280945A1.

The exemplary ANGPTL8 inhibitor used in the following Example is the human anti-ANGPTL8 antibody designated "H4H15341P". A heavy chain variable region (HCVR) comprising SEQ ID NO:1 and a light chain variable domain (LCVR) comprising SEQ ID NO:5; a heavy chain complementarity determining region 1 (HCDR1) comprising SEQ ID NO:2, a HCDR2 comprising SEQ ID NO:3, a HCDR3 comprising SEQ ID NO:4, a light chain complementarity determining region 1 (LCDR1) comprising SEQ ID NO:6, a LCDR2 comprising SEQ ID NO:7 and a LCDR3 comprising SEQ ID NO:8.

### Example 2. Generation of Human Antibodies to Human ANGPTL3

Human anti-ANGPTL3 antibodies were generated as described in US Patent No. 9,018,356. The exemplary ANGPTL3 inhibitor used in the following Example is the human anti-ANGPTL3 antibody designated "H4H1276S," also known as "evinacumab." H4H1276S has the following amino acid sequence characteristics: a heavy chain variable region (HCVR) comprising SEQ ID NO:10 and a light chain variable domain (LCVR) comprising SEQ ID NO:14; a heavy chain complementarity determining region 1 (HCDR1) comprising SEQ ID NO:11, a HCDR2 comprising SEQ ID NO:12, a HCDR3 comprising SEQ ID NO:13, a light chain complementarity determining region 1 (LCDR1) comprising SEQ ID NO:15, a LCDR2 comprising SEQ ID NO:16 and a LCDR3 comprising SEQ ID NO:17.

### Example 3: In Vivo Effect of Anti-ANGPTL3 Antibody on Circulating Triglyceride and Cholesterol Levels in ANGPTL8 Knockout (KO) and Wild Type (WT) Mice

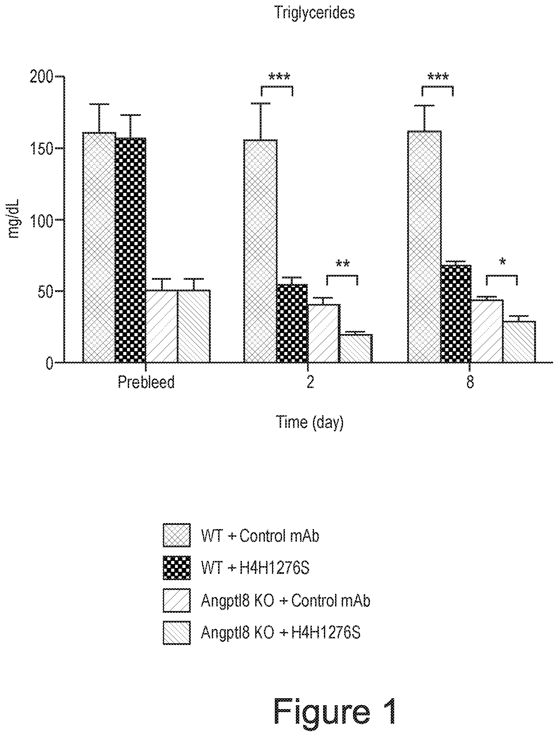

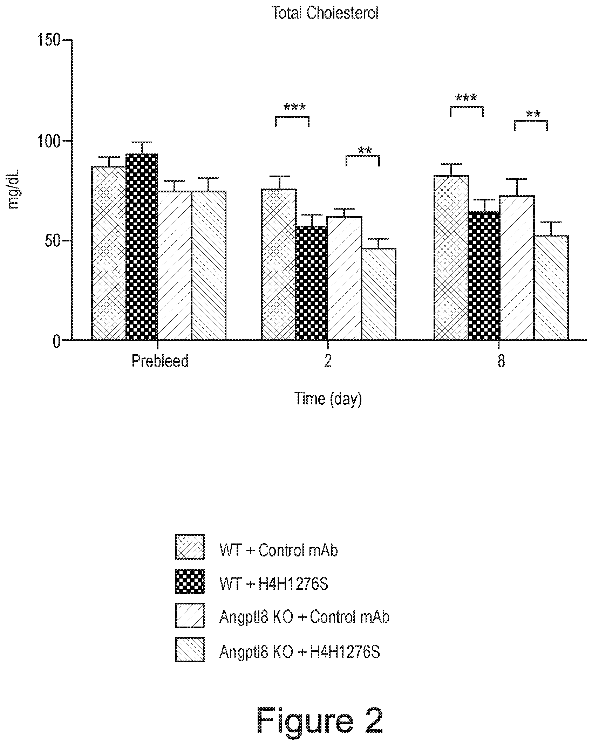

The effects of ANGPTL3 antibody H4H1276S on serum triglycerides (TG) and total cholesterol were evaluated in *Angptl8* knockout and wild-type mice. Mice were pre-bled at fasted-refed conditions (refed for 6 hours after overnight fast) 5 days before the experiment. The mice were sorted into groups (5 mice each per antibody per genotype) based on their TG and total cholesterol baseline values. The antibodies, isotype-matched (hlgG4) control with irrelevant specificity and H4H1276S (anti-ANGPTL3), were administered by single-dose subcutaneous injection on Day 0 of the study at 10mg/kg. Mice were bled at days 2 and 8 at fasted-refed conditions and TG and total cholesterol levels were determined in the serum by ADVIA® 1800 Chemistry System (Siemens). Averages were calculated for each time point. Results, expressed as (mean ± SEM) are shown in Figures 1 and 2. Figure 1 shows the results of triglyceride levels in both ANGPTL8 KO mice and WT mice. Figure 2 shows the results of total cholesterol levels in both ANGPTL8 KO mice and WT mice.

### Results summary:

The effect of ANGPTL3 antibody H4H1276S on circulating TG and total cholesterol levels was evaluated in *Angptl8* knockout and wild-type mice. H4H1276S treatment led to significant reduction in circulating TG and total cholesterol level in wild-type mice, consistent with that reported earlier (Gusarova et al, 2015, J. Lipid Res. (2015), Jul;56(7):1308-17). H4H1276S also caused significant reduction in TG levels in *Angptl8* knockout mice, which already had reduced baseline TG level due to *Angptl8* deletion. Total cholesterol was also significantly reduced by H4H1276S treatment of *Angptl8* knockout mice. These data suggest inhibition of both ANGPTL3 and ANGPTL8 may have an additive effect on circulating TG levels.

### Example 4: In Vivo Effect of Anti-ANGPTL8 and Anti-ANGPTL3 and their Combination on Circulating Triglycerides (TG) and Total Cholesterol (TC) in Humanized ANGPTL8 Mice

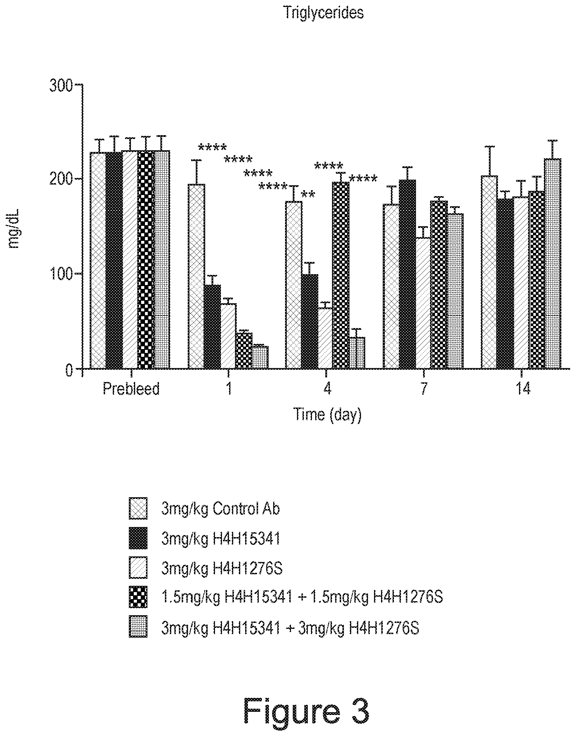

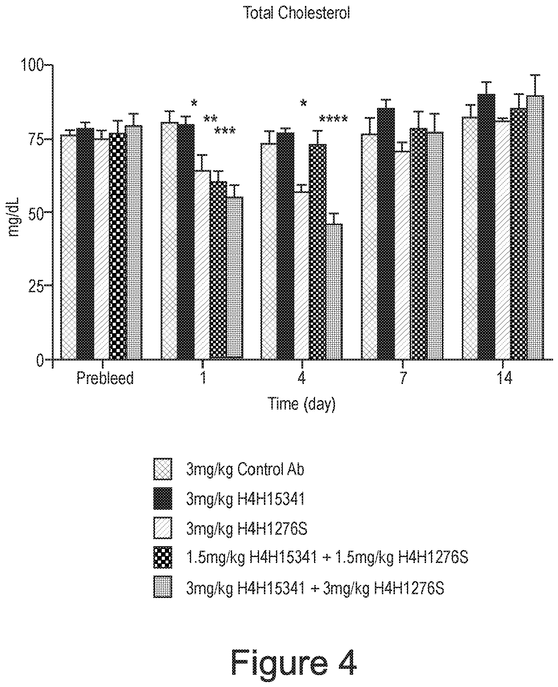

The effects of combinational treatment of anti-ANGPTL8 and anti-ANGPTL3 antibodies on serum triglycerides (TG) and total cholesterol levels were evaluated in humanized ANGPTL8 mice. Mice were pre-bled 6 days before the experiment at nonfasted conditions. The mice were sorted into groups (five mice each for each antibody or antibody combination tested) based on their baseline TG and body weights values. The antibodies were administered by single-dose subcutaneous injection on Day 0 of the study: isotype-matched (hlgG4) control with irrelevant specificity, H4H15341P and H4H1276S were administered at 3mg/kg dose and the combination of H4H15341P and H4H1276S were administered at 2 doses - 1.5mg/kg or 3mg/kg of each. Mice were bled (nonfasted) at days 1, 4, 8 and 14 after the antibodies injection, TG and total cholesterol levels were determined in the serum by ADVIA® 1800 Chemistry System (Siemens). Averages were calculated for each time point. Results, expressed as (mean ± SEM) are shown in Figures 3 and 4. Figure 3 shows the effect on triglycerides when the ANGPTL3 or ANGPTL8 antibodies were used alone, or when they were used in combination. Measurements were made at days 1, 4, 7 and 14 after administration. Figure 4 shows the effect on total cholesterol level when the ANGPTL3 or ANGPTL8 antibodies were used alone, or when they were used in combination. Measurements were made at days 1, 4, 7 and 14 after administration.

### Results summary:

The effect of combinational treatment of H4H15341P (anti-hANGPTL8) and H4H1276S (anti-ANGPTL3) on circulating TG levels were tested in humanized ANGPTL8 mice. The administration of single mAb or combination led to significant reduction in circulating TG level where combinational treatment shown an additive effect on serum TG levels compared to the single mAb effects.

### Example 5: Studies Regarding the Interaction of AngPTL3 and AngPTL8

Angiopoietin-like protein 3 (ANGPTL3) and ANGPTL8 are secreted proteins and inhibitors of lipoprotein lipase (LPL)-mediated plasma triglyceride clearance. How these ANGPTL proteins interact to regulate LPL activity to supply appropriate amounts of fatty acids to tissues for storage or oxidation was studied. ANGPTL3 inhibits LPL activity and increase serum triglycerides (TG) in mice independent of ANGPTL8. The effects on LPL activity and serum TG could be reversed with an ANGPTL3 blocking antibody. It was found that ANGPTL8 has a functional LPL inhibitory motif but requires ANGPTL3 to inhibit LPL and increase serum TG in mice. Site-directed mutagenesis revealed that the ability of ANGPTL8 to block LPL activity and increase serum TG did not require ANGPTL3 with a functional LPL inhibitory motif. An antibody to the C-terminus of ANGPTL8 (see Example 6) reversed LPL inhibition by ANGPTL8 in the presence of ANGPTL3. The antibody did not disrupt the ANGPTL8:ANGPTL3 complex but comes in close proximity to the LPL inhibitory motif in the N-terminus of ANGPTL8. Collectively, these data show that ANGPTL8 has a functional LPL inhibitory motif but can only inhibit LPL in the presence of ANGPTL3 (Haller, J. et al. Nature Scientific Reports, submitted 2017).

### Example 6: Anti-ANGPTL8 blocking antibody reverses ANGPTL8-induced inhibition of LPL without disrupting the ANGPTL3 interaction

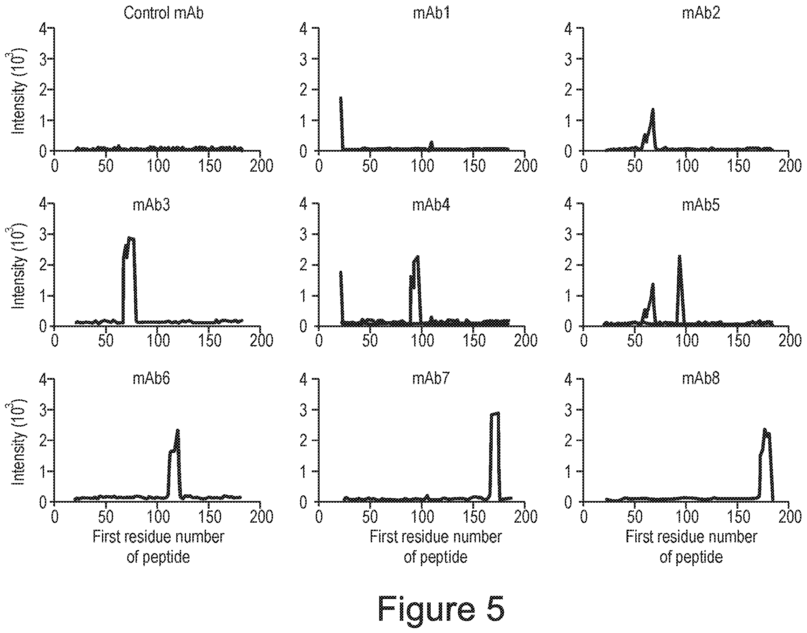

A library of peptides of 15 amino acids in length and with an offset of one residue was used to identify epitopes for 8 monoclonal antibodies that bind human ANGPTL8 with high affinity (K_{d} = 2.4x10⁻¹⁰ to 5.8x10⁻⁹ M; Figure 5). The epitopes are depicted in Fig. 6a. The antibodies do not cross react to mouse ANGPTL8 and were tested for *in vivo* efficacy in humanized ANGPTL8 mice (Gusarova et al., Submitted 2017). Surprisingly, only one antibody (mAb 7 or H4H15341P) produced a significant reduction in serum TG (Fig. 6b). This antibody binds to an epitope (amino acids 171-180) in the C-terminal region of ANGPTL8 (Fig. 6a). We have recently reported that this antibody also lowers circulating TG by 65% in cynomolgus monkeys (Gusarova et al., Submitted 2017). Fig. 6c shows that the antibody dose-dependently (EC₅₀=0.47 nM) reversed the inhibitory effect of ANGPTL8 on LPL. This study was performed in HEK293 cells co-expressing ANGPTL3.

Since the anti-ANGPTL8 blocking antibody binds to the C-terminal part and the LPL inhibitory motif is located in the N-terminal region, we wanted to test if it mediates its action by disrupting the interaction of ANGPTL3 and ANGPTL8. To this end, we used an AlphaLISA proximity assay where a signal is detected when ANGPTL3 and ANGPTL8 are expressed together (Fig. 6d, left). The AlphaLISA signal remained strong even when high concentrations of the antibody were tested. These data indicate that the anti-ANGPTL8 blocking antibody does not restore LPL activity by disrupting the ANGPTL8:ANGPTL3 interaction.

Structural modeling has recently predicted that ANGPTL8 folds in a manner so that the N- and C-terminal domains are in close proximity (Siddiqa et al. (2016) Comput Biol Chem, 61, 210-220). Thus, we hypothesized that the ANGPTL8 blocking antibody may sterically shield the inhibitory motif in ANGPTL8 to prevent LPL inhibition. To test this hypothesis, we labeled the ANGPTL8 blocking antibody and a non-blocking antibody to ANGPTL8 (mAb4 (H4H15347P)), which binds to the mid region of ANGPTL8 protein sequence, with a FRET acceptor dye. In addition, we labeled ANGPTL8 in its N-terminal site with a donor dye as depicted in Fig. 6e. When labeled ANGPTL8 was co-expressed with ANGPTL3 in HEK293 cells and assayed with the labeled antibodies, we detected a much stronger TR-FRET signal with the ANGPTL8 blocking antibody directed to C-terminal fragment compared to that of the non-blocking antibody (Fig. 6f). These results indicate that the N- and C-termini of ANGPTL8 are located in close proximity and that the ANGPTL8 blocking antibody may sterically prevent binding of the ANGPTL8 inhibitory motif to LPL.

### Materials and Methods:

**Epitope mapping.** Epitope mapping was performed by PEPSCAN PRESTO BE. Briefly, standard Fmoc-peptide synthesis was used to synthesize 15-amino acid long peptides with 14 amino acid overlap. Antibody binding to each peptide was tested in a PEPSCAN-based ELISA: primary antibody was incubated at 1 µg/ml (overnight at 4°C) followed by incubation with goat anti-human HRP conjugate (1 h at 25°C). After washing, the peroxidase substrate 2,2'-azino-di-3ethylbenzthiazoline sulfonate (ABTS) and 10 µl/ml of 3% H₂O₂ were added and color development was measured using a charge couple device camera.

**AlphaLISA assay**. Assay was performed in a 384-well plate format at room temperature. CHO-K1 cell media (2.5 µl) from cells transfected with a vector containing ANGPTL3-myc and ANGPTL8-V5 or empty vector was incubated with the indicated amounts of antibodies for 1h in a final volume of 20 µl. Then anti-V5 Alpha acceptor beads (PerkinElmer) were added and incubated for an additional 30 min, follow by 1 h incubation with biotinylated anti-myc antibody (generated in house) and streptavidin Alpha donor beads (PerkinElmer). All dilutions were performed in Hi-Block buffer (PerkinElmer), final concentration was 10 ng/ml for beads and 10 nM for biotinylated antibody in total volume of 50 µl. AlphaLISA signal was measured in an Envision® Multilabel Reader (PerkinElmer) following manufacturer's instructions.

**TR-FRET antibody experiment.** HEK293T cells were co-transfected with a plasmid coding for ANGPTL3 and a plasmid coding for N-terminal Avi-tagged ANGPTL8 using TranslT-LT1 transfection reagent. Cell media was collected after 72 h, concentrated 20x using Centriprep (Millipore) filter unit with a 10 kDa molecular cutoff, and site direct biotinylated using biotin-protein ligase BirA (Avidity) (Fairhead & Howarth, 2015) following supplier instructions, then dialyzed against PBS. Biotinylation of Avi-ANGPTL8 was confirmed by Western blot using streptavidin-HRP for detection. Antibodies were labeled using Alexa FluorTM 647 Antibody Labeling Kit (Invitrogen). Antibody and dye concentrations were determined spectrophotometrically and drug to antibody ratios of 5:2 were used for all experiments. TR-FRET was performed in a 384-well plate format. Final concentration were 31.3 nM Europium-streptavidin, 25 nM labeled antibody, 50% Biotin-Avi-ANGPTL8 in TR-FRET dilution buffer (PerkinElmer). TR-FRET was measured using Envision® Multilabel Reader (PerkinElmer), excitation filter 340/30 nm, emission filter 1 - 615/8.5 nm, emission filter 2 - 665/7.5 nm, dichroic mirror D400/D630, measured in a delay of 100 ms and a window time of 2 ms.

## Claims

1. An angiopoietin-like protein 8 (ANGPTL8) inhibitor for use in a method of treating a patient suffering from hyperlipidemia in combination with an inhibitor of angiopoietin-like protein 3 (ANGPTL3), wherein the ANGPTL8 inhibitor is an antibody or an antigen-binding fragment thereof that binds specifically to ANGPTL8 and comprises a heavy chain CDR1 (HCDR1) having the amino acid sequence of SEQ ID NO: 2, a HCDR2 having the amino acid sequence of SEQ ID NO: 3, a HCDR3 having the amino acid sequence of SEQ ID NO: 4, a light chain CDR1 (LCDR1) having the amino acid sequence of SEQ ID NO: 6, a LCDR2 having the amino acid sequence of SEQ ID NO: 7, and a LCDR3 having the amino acid sequence of SEQ ID NO: 8; and the ANGPTL3 inhibitor is an antibody or an antigen-binding fragment thereof that binds specifically to ANGPTL3 and comprises a HCDR1 having the amino acid sequence of SEQ ID NO: 11, a HCDR2 having the amino acid sequence of SEQ ID NO: 12, a HCDR3 having the amino acid sequence of SEQ ID NO: 13, a LCDR1 having the amino acid sequence of SEQ ID NO: 15, a LCDR2 having the amino acid sequence of SEQ ID NO: 16, and a LCDR3 having the amino acid sequence of SEQ ID NO: 17.

2. An inhibitor of angiopoietin-like protein 3 (ANGPTL3) for use in a method of treating a patient suffering from hyperlipidemia in combination with an angiopoietin-like protein 8 (ANGPTL8) inhibitor, wherein the ANGPTL3 inhibitor is an antibody or an antigen-binding fragment thereof that binds specifically to ANGPTL3 and comprises a heavy chain CDR1 (HCDR1) having the amino acid sequence of SEQ ID NO: 11, a HCDR2 having the amino acid sequence of SEQ ID NO: 12, a HCDR3 having the amino acid sequence of SEQ ID NO: 13, a light chain CDR1 (LCDR1) having the amino acid sequence of SEQ ID NO: 15, a LCDR2 having the amino acid sequence of SEQ ID NO: 16, and a LCDR3 having the amino acid sequence of SEQ ID NO: 17; and the ANGPTL8 inhibitor is an antibody or an antigen-binding fragment thereof that binds specifically to ANGPTL8 and comprises a HCDR1 having the amino acid sequence of SEQ ID NO: 2, a HCDR2 having the amino acid sequence of SEQ ID NO: 3, a HCDR3 having the amino acid sequence of SEQ ID NO: 4, a LCDR1 having the amino acid sequence of SEQ ID NO: 6, a LCDR2 having the amino acid sequence of SEQ ID NO: 7, and a LCDR3 having the amino acid sequence of SEQ ID NO: 8.

3. A pharmaceutical composition for use in a method of treating a patient suffering from hyperlipidemia, wherein the composition comprises a therapeutically effective amount of a combination of an angiopoietin-like protein 8 (ANGPTL8) inhibitor and an inhibitor of angiopoietin-like protein 3 (ANGPTL3), wherein the ANGPTL8 inhibitor is an antibody or an antigen-binding fragment thereof that binds specifically to ANGPTL8 and comprises a heavy chain CDR1 (HCDR1) having the amino acid sequence of SEQ ID NO: 2, a HCDR2 having the amino acid sequence of SEQ ID NO: 3, a HCDR3 having the amino acid sequence of SEQ ID NO: 4, a light chain CDR1 (LCDR1) having the amino acid sequence of SEQ ID NO: 6, a LCDR2 having the amino acid sequence of SEQ ID NO: 7, and a LCDR3 having the amino acid sequence of SEQ ID NO: 8; and the ANGPTL3 inhibitor is an antibody or an antigen-binding fragment thereof that binds specifically to ANGPTL3 and comprises a HCDR1 having the amino acid sequence of SEQ ID NO: 11, a HCDR2 having the amino acid sequence of SEQ ID NO: 12, a HCDR3 having the amino acid sequence of SEQ ID NO: 13, a LCDR1 having the amino acid sequence of SEQ ID NO: 15, a LCDR2 having the amino acid sequence of SEQ ID NO: 16, and a LCDR3 having the amino acid sequence of SEQ ID NO: 17.

4. The ANGPTL8 inhibitor for use in the method of claim 1, the inhibitor of ANGPTL3 for use in the method of claim 2, or the pharmaceutical composition for use in the method of claim 3, wherein:
(a) the hyperlipidemia is familial hyperlipidemia or acquired hyperlipidemia, optionally wherein (i) the familial hyperlipidemia is hypercholesterolemia selected from the group consisting of heterozygous familial hypercholesterolemia (HeFH) and homozygous familial hypercholesterolemia (HoFH) or (ii) the acquired hyperlipidemia is the result of excessive drinking of alcohol, obesity, side effects of medications (e.g. hormones or steroids), diabetes, kidney disease, underactive thyroid gland, or pregnancy; or
(b) the hyperlipidemia is selected from the group consisting of hyperlipoproteinemia, hypercholesterolemia, hypertriglyceridemia, and hyperchylomicronemia.

5. The ANGPTL8 inhibitor for use in the method of claim 1, the inhibitor of ANGPTL3 for use in the method of claim 2, or the pharmaceutical composition for use in the method of claim 3, wherein the antibody, or antigen-binding fragment thereof, that binds specifically to ANGPTL8 comprises a HCVR having the amino acid sequence of SEQ ID NO: 1 and a LCVR having the amino acid sequence of SEQ ID NO: 5.

6. The ANGPTL8 inhibitor for use in the method of claim 1, or the inhibitor of ANGPTL3 for use in the method of claim 2, wherein the ANGPTL8 inhibitor is administered to the patient subcutaneously or intravenously.

7. The ANGPTL8 inhibitor for use in the method of claim 1, the inhibitor of ANGPTL3 for use in the method of claim 2, or the pharmaceutical composition for use in the method of claim 3, wherein:
(a) the antibody, or antigen-binding fragment thereof, that binds specifically to ANGPTL3 comprises a HCVR having the amino acid sequence of SEQ ID NO: 10 and a LCVR having the amino acid sequence of SEQ ID NO: 14; and/or
(b) the ANGPTL3 antibody is evinacumab.

8. The ANGPTL8 inhibitor for use in the method of claim 1, or the inhibitor of ANGPTL3 for use in the method of claim 2, wherein the ANGPTL3 inhibitor is administered to the patient subcutaneously or intravenously.

9. The ANGPTL8 inhibitor for use in the method of claim 1, or the inhibitor of ANGPTL3 for use in the method of claim 2, wherein
(a) the ANGPTL8 inhibitor and the ANGPTL3 inhibitor are administered concurrently or sequentially; or
(b) the ANGPTL8 inhibitor and the ANGPTL3 inhibitor are administered at therapeutically effective concentrations in separate pharmaceutical compositions or are co-formulated in one pharmaceutical composition.

10. The pharmaceutical composition for use in the method of claim 3, wherein the pharmaceutical composition is administered to the patient subcutaneously or intravenously.

## Patentansprüche

1. Inhibitor des Angiopoietin-ähnlichen Proteins 8 (ANGPTL8) für die Verwendung in einem Verfahren zum Behandeln eines Patienten, der an Hyperlipidämie leidet, in Kombination mit einem Inhibitor des Angiopoietin-ähnlichen Proteins 3 (ANGPTL3), wobei der ANGPTL8-Inhibitor ein Antikörper oder ein Antigen-bindendes Fragment davon ist, der/das spezifisch an ANGPTL8 bindet und eine schwere Kette CDR1 (HCDR1) mit der Aminosäuresequenz von SEQ ID NO: 2, eine HCDR2 mit der Aminosäuresequenz von SEQ ID NO: 3, eine HCDR3 mit der Aminosäuresequenz von SEQ ID NO: 4, eine leichte Kette CDR1 (LCDR1) mit der Aminosäuresequenz von SEQ ID NO: 6, eine LCDR2 mit der Aminosäuresequenz von SEQ ID NO: 7, und eine LCDR3 mit der Aminosäuresequenz von SEQ ID NO: 8 umfasst; und der ANGPTL3-Inhibitor ein Antikörper oder ein Antigen-bindendes Fragment davon ist, der/das spezifisch an ANGPTL3 bindet und eine HCDR1 mit der Aminosäuresequenz von SEQ ID NO: 11, eine HCDR2 mit der Aminosäuresequenz von SEQ ID NO: 12, eine HCDR3 mit der Aminosäuresequenz von SEQ ID NO: 13, eine LCDR1 mit der Aminosäuresequenz von SEQ ID NO: 15, eine LCDR2 mit der Aminosäuresequenz von SEQ ID NO: 16, und eine LCDR3 mit der Aminosäuresequenz von SEQ ID NO: 17 umfasst.

2. Inhibitor des Angiopoietin-ähnlichen Proteins 3 (ANGPTL3) für die Verwendung in einem Verfahren zum Behandeln eines Patienten, der an Hyperlipidämie leidet, in Kombination mit einem Inhibitor des Angiopoietin-ähnlichen Proteins 8 (ANGPTL8), wobei der ANGPTL3-Inhibitor ein Antikörper oder ein Antigen-bindendes Fragment davon ist, der/das spezifisch an ANGPTL3 bindet und eine schwere Kette CDR1 (HCDR1) mit der Aminosäuresequenz von SEQ ID NO: 11, eine HCDR2 mit der Aminosäuresequenz von SEQ ID NO: 12, eine HCDR3 mit der Aminosäuresequenz von SEQ ID NO: 13, eine leichte Kette CDR1 (LCDR1) mit der Aminosäuresequenz von SEQ ID NO: 15, eine LCDR2 mit der Aminosäuresequenz von SEQ ID NO: 16, und eine LCDR3 mit der Aminosäuresequenz von SEQ ID NO: 17 umfasst; und der ANGPTL8-Inhibitor ein Antikörper oder ein Antigen-bindendes Fragment davon ist, der/das spezifisch an ANGPTL8 bindet und eine HCDR1 mit der Aminosäuresequenz von SEQ ID NO: 2, eine HCDR2 mit der Aminosäuresequenz von SEQ ID NO: 3, eine HCDR3 mit der Aminosäuresequenz von SEQ ID NO: 4, eine LCDR1 mit der Aminosäuresequenz von SEQ ID NO: 6, eine LCDR2 mit der Aminosäuresequenz von SEQ ID NO: 7, und eine LCDR3 mit der Aminosäuresequenz von SEQ ID NO: 8 umfasst.

3. Pharmazeutische Zusammensetzung für die Verwendung in einem Verfahren zum Behandeln eines Patienten, der an Hyperlipidämie leidet, wobei die Zusammensetzung eine therapeutisch wirksame Menge einer Kombination aus einem Inhibitor des Angiopoietin-ähnlichen Proteins 8 (ANGPTL8) und einem Inhibitor des Angiopoietin-ähnlichen Proteins 3 (ANGPTL3) umfasst, wobei der ANGPTL8-Inhibitor ein Antikörper oder ein Antigen-bindendes Fragment davon ist, der/das spezifisch an ANGPTL8 bindet und eine schwere Kette CDR1 (HCDR1) mit der Aminosäuresequenz von SEQ ID NO: 2, eine HCDR2 mit der Aminosäuresequenz von SEQ ID NO: 3, eine HCDR3 mit der Aminosäuresequenz von SEQ ID NO: 4, eine leichte Kette CDR1 (LCDR1) mit der Aminosäuresequenz von SEQ ID NO: 6, eine LCDR2 mit der Aminosäuresequenz von SEQ ID NO: 7, und eine LCDR3 mit der Aminosäuresequenz von SEQ ID NO: 8 umfasst; und der ANGPTL3-Inhibitor ein Antikörper oder ein Antigen-bindendes Fragment davon ist, der/das spezifisch an ANGPTL3 bindet und eine HCDR1 mit der Aminosäuresequenz von SEQ ID NO: 11, eine HCDR2 mit der Aminosäuresequenz von SEQ ID NO: 12, eine HCDR3 mit der Aminosäuresequenz von SEQ ID NO: 13, eine LCDR1 mit der Aminosäuresequenz von SEQ ID NO: 15, eine LCDR2 mit der Aminosäuresequenz von SEQ ID NO: 16, und eine LCDR3 mit der Aminosäuresequenz von SEQ ID NO: 17 umfasst.

4. ANGPTL8-Inhibitor für die Verwendung in dem Verfahren nach Anspruch 1, ANGPTL3-Inhibitor für die Verwendung in dem Verfahren nach Anspruch 2 oder pharmazeutische Zusammensetzung für die Verwendung in dem Verfahren nach Anspruch 3, wobei:
(a) die Hyperlipidämie eine familiäre Hyperlipidämie oder eine erworbene Hyperlipidämie ist, optional wobei (i) die familiäre Hyperlipidämie eine Hypercholesterinämie ist, die aus der Gruppe ausgewählt ist, die aus heterozygoter familiärer Hypercholesterinämie (HeFH) und homozygoter familiärer Hypercholesterinämie (HoFH) besteht, oder (ii) die erworbene Hyperlipidämie das Ergebnis von übermäßigem Alkoholkonsum, Fettleibigkeit, Nebenwirkungen von Medikamenten (z. B. Hormone oder Steroide), Diabetes, Nierenerkrankungen, Schilddrüsenunterfunktion oder Schwangerschaft ist; oder
(b) die Hyperlipidämie aus der Gruppe ausgewählt ist, die aus Hyperlipoproteinämie, Hypercholesterinämie, Hypertriglyceridämie und Hyperchylomikronämie besteht.

5. ANGPTL8-Inhibitor für die Verwendung in dem Verfahren nach Anspruch 1, ANGPTL3-Inhibitor für die Verwendung in dem Verfahren nach Anspruch 2 oder pharmazeutische Zusammensetzung für die Verwendung in dem Verfahren nach Anspruch 3, wobei der Antikörper oder das Antigen-bindende Fragment davon, der/das spezifisch an ANGPTL8 bindet, eine HCVR mit der Aminosäuresequenz von SEQ ID NO: 1 und eine LCVR mit der Aminosäuresequenz von SEQ ID NO: 5 umfasst.

6. ANGPTL8-Inhibitor für die Verwendung in dem Verfahren nach Anspruch 1 oder ANGPTL3-Inhibitor für die Verwendung in dem Verfahren nach Anspruch 2, wobei der ANGPTL8-Inhibitor dem Patienten subkutan oder intravenös verabreicht wird.

7. ANGPTL8-Inhibitor für die Verwendung in dem Verfahren nach Anspruch 1, ANGPTL3-Inhibitor für die Verwendung in dem Verfahren nach Anspruch 2 oder pharmazeutische Zusammensetzung für die Verwendung in dem Verfahren nach Anspruch 3, wobei:
(a) der Antikörper oder das Antigen-bindende Fragment davon, der/das spezifisch an ANGPTL3 bindet, eine HCVR mit der Aminosäuresequenz von SEQ ID NO: 10 und eine LCVR mit der Aminosäuresequenz von SEQ ID NO: 14 umfasst; und/oder
(b) der ANGPTL3-Antikörper Evinacumab ist.

8. ANGPTL8-Inhibitor für die Verwendung in dem Verfahren nach Anspruch 1 oder ANGPTL3-Inhibitor für die Verwendung in dem Verfahren nach Anspruch 2, wobei der ANGPTL3-Inhibitor dem Patienten subkutan oder intravenös verabreicht wird.

9. ANGPTL8-Inhibitor für die Verwendung in dem Verfahren nach Anspruch 1 oder ANGPTL3-Inhibitor für die Verwendung in dem Verfahren nach Anspruch 2, wobei
(a) der ANGPTL8-Inhibitor und der ANGPTL3-Inhibitor gleichzeitig oder nacheinander verabreicht werden; oder
(b) der ANGPTL8-Inhibitor und der ANGPTL3-Inhibitor in therapeutisch wirksamen Konzentrationen in getrennten pharmazeutischen Zusammensetzungen verabreicht werden oder in einer einzigen pharmazeutischen Zusammensetzung co-formuliert sind.

10. Pharmazeutische Zusammensetzung für die Verwendung in dem Verfahren nach Anspruch 3, wobei die pharmazeutische Zusammensetzung dem Patienten subkutan oder intravenös verabreicht wird.

## Revendications

1. Inhibiteur de protéine 8 du type angiopoïétine (ANGPTL8) à utiliser dans un procédé de traitement d'un patient souffrant d'hyperlipidémie en association avec un inhibiteur de la protéine 3 du type angiopoïétine (ANGPTL3), dans lequel l'inhibiteur d'ANGPTL8 est un anticorps ou un fragment de liaison à l'antigène de celui-ci qui se lie spécifiquement à ANGPTL8 et comprend une CDR1 à chaîne lourde (HCDR1) ayant la séquence d'acides aminés de SEQ ID NO : 2, une HCDR2 ayant la séquence d'acides aminés de SEQ ID NO : 3, une HCDR3 ayant la séquence d'acides aminés de SEQ ID NO : 4, une CDR1 à chaîne légère (LCDR1) ayant la séquence d'acides aminés de SEQ ID NO : 6, une LCDR2 ayant la séquence d'acides aminés de SEQ ID NO : 7, et une LCDR3 ayant la séquence d'acides aminés de SEQ ID NO : 8 ; et l'inhibiteur d'ANGPTL3 est un anticorps ou un fragment de liaison à l'antigène de celui-ci qui se lie spécifiquement à la ANGPTL3 et comprend une HCDR1 ayant la séquence d'acides aminés de SEQ ID NO : 11, une HCDR2 ayant la séquence d'acides aminés de SEQ ID NO : 12, une HCDR3 ayant la séquence d'acides aminés de SEQ ID NO : 13, une LCDR1 ayant la séquence d'acides aminés de SEQ ID NO : 15, une LCDR2 ayant la séquence d'acides aminés de SEQ ID NO : 16, et une LCDR3 ayant la séquence d'acides aminés de SEQ ID NO : 17.

2. Inhibiteur de la protéine 3 du type angiopoïétine (ANGPTL3) à utiliser dans un procédé de traitement d'un patient souffrant d'hyperlipidémie en association avec un inhibiteur de la protéine 8 du type angiopoïétine (ANGPTL8), dans lequel l'inhibiteur d'ANGPTL3 est un anticorps ou un fragment de liaison à l'antigène de celui-ci qui se lie spécifiquement à ANGPTL3 et comprend une CDR1 à chaîne lourde (HCDR1) ayant la séquence d'acides aminés de SEQ ID NO : 11, une HCDR2 ayant la séquence d'acides aminés de SEQ ID NO : 12, une HCDR3 ayant la séquence d'acides aminés de SEQ ID NO : 13, une CDR1 à chaîne légère (LCDR1) ayant la séquence d'acides aminés de SEQ ID NO : 15, une LCDR2 ayant la séquence d'acides aminés de SEQ ID NO : 16, et une LCDR3 ayant la séquence d'acides aminés de SEQ NO : 17 ; et l'inhibiteur d'ANGPTL8 est un anticorps ou un fragment de liaison à l'antigène de celui-ci qui se lie spécifiquement à ANGPTL8 et comprend une HCDR1 ayant la séquence d'acides aminés de SEQ ID NO : 2, une HCDR2 ayant la séquence d'acides aminés de SEQ ID NO : 3, une HCDR3 ayant la séquence d'acides aminés de SEQ ID NO : 4, une LCDR1 ayant la séquence d'acides aminés de SEQ ID NO : 6, une LCDR2 ayant la séquence d'acides aminés de SEQ ID NO : 7, et une LCDR3 ayant la séquence d'acides aminés de SEQ ID NO : 8.

3. Composition pharmaceutique à utiliser dans un procédé de traitement d'un patient souffrant d'hyperlipidémie, dans laquelle la composition comprend une quantité thérapeutiquement efficace d'une combinaison d'un inhibiteur de la protéine 8 du type angiopoïétine (ANGPTL8) et d'un inhibiteur de la protéine 3 du type angiopoïétine (ANGPTL3), dans laquelle l'inhibiteur d'ANGPTL8 est un anticorps ou un fragment de liaison à l'antigène de celui-ci qui se lie spécifiquement à ANGPTL8 et comprend une CDR1 à chaîne lourde (HCDR1) ayant la séquence d'acides aminés de SEQ ID NO : 2, une HCDR2 ayant la séquence d'acides aminés de SEQ ID NO : 3, une HCDR3 ayant la séquence d'acides aminés de SEQ ID NO : 4, une CDR1 à chaîne légère (LCDR1) ayant la séquence d'acides aminés de SEQ ID NO : 6, une LCDR2 ayant la séquence d'acides aminés de SEQ ID NO : 7, et une LCDR3 ayant la séquence d'acides aminés de SEQ ID NO : 8 ; et l'inhibiteur d'ANGPTL3 est un anticorps ou un fragment de liaison à l'antigène de celui-ci qui se lie spécifiquement à ANGPTL3 et comprend une HCDR1 ayant la séquence d'acides aminés de SEQ ID NO : 11, une HCDR2 ayant la séquence d'acides aminés de SEQ ID NO : 12, une HCDR3 ayant la séquence d'acides aminés de SEQ ID NO : 13, une LCDR1 ayant la séquence d'acides aminés de SEQ ID NO : 15, une LCDR2 ayant la séquence d'acides aminés de SEQ ID NO : 16, et une LCDR3 ayant la séquence d'acides aminés de SEQ ID NO : 17.

4. Inhibiteur d'ANGPTL8 à utiliser dans le procédé de la revendication 1, l'inhibiteur d'ANGPTL3 à utiliser dans le procédé de la revendication 2, ou la composition pharmaceutique à utiliser dans le procédé de la revendication 3, dans lesquels :
(a) l'hyperlipidémie est une hyperlipidémie familiale ou une hyperlipidémie acquise, éventuellement dans lesquels (i) l'hyperlipidémie familiale est une hypercholestérolémie choisie dans le groupe constitué de l'hypercholestérolémie familiale hétérozygote (HeFH) et de l'hypercholestérolémie familiale homozygote (HoFH) ou (ii) l'hyperlipidémie acquise est le résultat d'une consommation excessive d'alcool, d'obésité, d'effets secondaires des médicaments (par exemple, hormones ou stéroïdes), de diabète, de maladie rénale, d'hypothyroïdie ou de grossesse ; ou
(b) l'hyperlipidémie est choisie dans le groupe constitué par l'hyperlipoprotéinémie, l'hypercholestérolémie, l'hypertriglycéridémie et l'hyperchylomicronémie.

5. Inhibiteur d'ANGPTL8 à utiliser dans le procédé de la revendication 1, l'inhibiteur d'ANGPTL3 à utiliser dans le procédé de la revendication 2, ou la composition pharmaceutique à utiliser dans le procédé de la revendication 3, dans lesquels l'anticorps, ou un fragment de liaison à l'antigène de celui-ci, qui se lie spécifiquement à ANGPTL8 comprend une HCVR ayant la séquence d'acides aminés de SEQ ID NO : 1 et une LCVR ayant la séquence d'acides aminés de SEQ ID NO : 5.

6. Inhibiteur d'ANGPTL8 à utiliser dans le procédé de la revendication 1, ou l'inhibiteur d'ANGPTL3 à utiliser dans le procédé de la revendication 2, dans lesquels l'inhibiteur d'ANGPTL8 est administré au patient par voie sous-cutanée ou intraveineuse.

7. Inhibiteur d'ANGPTL8 à utiliser dans le procédé de la revendication 1, l'inhibiteur d'ANGPTL3 à utiliser dans le procédé de la revendication 2, ou la composition pharmaceutique à utiliser dans le procédé de la revendication 3, dans lesquels :
(a) l'anticorps ou le fragment de liaison à l'antigène de celui-ci qui se lie spécifiquement à ANGPTL3 comprend une HCVR ayant la séquence d'acides aminés de SEQ ID NO : 10 et une LCVR ayant la séquence d'acides aminés de SEQ ID NO : 14 ; et/ou
(b) l'anticorps d'ANGPTL3 est l'evinacumab.

8. Inhibiteur d'ANGPTL8 à utiliser dans le procédé de la revendication 1, ou inhibiteur d'ANGPTL3 à utiliser dans le procédé de la revendication 2, dans lesquels l'inhibiteur d'ANGPTL3 est administré au patient par voie sous-cutanée ou intraveineuse.

9. Inhibiteur d'ANGPTL8 à utiliser dans le procédé de la revendication 1, ou l'inhibiteur d'ANGPTL3 à utiliser dans le procédé de la revendication 2, dans lesquels
(a) l'inhibiteur d'ANGPTL8 et l'inhibiteur d'ANGPTL3 sont administrés simultanément ou séquentiellement ; ou
(b) l'inhibiteur d'ANGPTL8 et l'inhibiteur d'ANGPTL3 sont administrés à des concentrations thérapeutiquement efficaces dans des compositions pharmaceutiques séparées ou sont co-formulés dans une composition pharmaceutique.

10. Composition pharmaceutique à utiliser dans le procédé de la revendication 3, dans laquelle la composition pharmaceutique est administrée au patient par voie sous-cutanée ou intraveineuse.
